# EUROPEAN PATENT APPLICATION

(11) **EP 3 940 704 A1**
(43) Date of publication of application: **19.01.2022**
(21) Application number: 20185693.7
(22) Date of filing: 14.07.2020
(51) Int. Cl.: G16B 25/30, G16B 5/00, G16B 5/20, G16B 20/00

(54) **METHOD FOR DETERMINING THE DIFFERENTIATION STATE OF A STEM CELL**

(71) Applicant: Koninklijke Philips N.V., 5656 AG Eindhoven (NL)
(72) Inventor: VAN DE STOLPE, Anja, 5656 AE Eindhoven (NL); HOLTZER, Laurentius Henricus Franciscus Maria, 5656 AE Eindhoven (NL)
(74) Representative: Philips Intellectual Property & Standards

(57) **Abstract**

The present invention relates to a method for determining the differentiation state of a stem cell, based on cellular signaling pathway activities. The method can be used to determine the pluripotency, multipotency or unipotency of a stem cell. The invention further relates to a method for generating a reference library for use in the method for determining the differentiation state. The invention further relates a non-transitory storage medium for executing the method, a kit of parts suitable for performing the method and use of the kit of parts in performing the method of the invention.

## Description

### FIELD OF THE INVENTION

The present invention relates to a method for determining the differentiation state of a stem cell. The method further relates to a method for generating a reference library, for use in a method for characterizing a stem cell. Further the invention relates to a kit suitable for use in the methods described herein.

### BACKGROUND OF THE INVENTION

Coordinated activity of a relatively small number of signal transduction pathways control embryonic development, and therefore the cell division, migration and differentiation of stem cells with varying differentiation potential. The signaling pathways are evolutionary well conserved and control these basic cellular mechanisms.

Signaling pathways can be roughly categorized as hormonal driven nuclear receptor pathways (e.g. androgen and estrogen receptor pathways), developmental pathways (e.g. Wnt, Hedgehog, TGFβ, Notch pathways), the inflammatory NFκB pathway, and the highly complex growth factor regulated signaling pathway network, in which the PI3K-AKT-mTOR pathway is probably the most prominent one, next to MAPK and the JAK-STAT pathways. In addition to embryonic development they control important physiological processes, e.g. hematopoiesis and generation of an immune response, tissue regeneration, hair growth, and renewal of intestinal mucosa. In both physiological cellular processes as well as embryonic development their activity is tightly controlled.

Stem cell technologies have matured over the past decades, with the major disruptive discovery and subsequent development being the reliable generation of induced Pluripotent Stem cells (iPS). Developments in induced pluripotent stem cell (iPS) technology have resulted in robust protocols enabling the generation of a pluripotent stem cell line from every healthy or diseased individual [Chacón-Martínez, et al., Development, vol. 145, no. 15, p. dev165399, Aug. 2018]. However, other types of stem cells are also known, like human embryonic stem cells, organ stem cells, such as hematopoietic stem cells or intestinal crypt stem cells. While iPS cells and HES cells are pluripotent, meaning they can in principle form all the cell types of the human body, organ stem cells are mostly multipotent and can form multiple cell types that make up the organ tissue; progenitor stem cells in an organ can generally only form one mature cell type.

Culture of iPS cells and differentiation towards specific cell types is increasingly used to explore creation of in vitro disease models to gain knowledge on disease pathophysiology and for drug development purposes. iPS-based disease models are unique because the disease is generated within the genetic context of the donor individual. Aside from understanding disease pathophysiology and drug development, utility of iPS-based disease models may lie in a personalized disease model for patients with certain inherited diseases. This can be especially relevant in case it is difficult to obtain pathology material, for example for children with brain disorders like epilepsy and cognitive disorders. The iPS-derived disease model may in this case serve diagnostic purposes by enabling identification of the pathogenic defect and in vitro investigation of drug efficacy for that specific patient.

For simple genetic diseases a control "companion" an iPS cell line can be generated in which the mutation is corrected, facilitated by development of CRISP-CAS technology. For complex genetic diseases this is not possible. There the advantage of the iPS technology lies in the potential to collect an increasing number of iPS lines derived from patients with the same disease and recreate the disease in culture in a standardized manner. Such patient derived disease-models may then be used as surrogate patients "in a dish", for example for drug development purposes. Therefore there is a need for assays to ascertain that various used batches of stem cells have the same characteristics, to define the differentiation steps towards the cell types required for mimicking the disease, and to assess effects of for example addition of drugs to the cell culture disease model.

Stem cells enable in vitro culture of differentiated cells or tissue to return to the patient for organ or tissue repair. While human embryonic stem cells (HES) are used to explore some regenerative medicine applications, iPS cell lines in general have more potential for use in regenerative medicine [Chacón-Martínez, et al., Development, vol. 145, no. 15, p. dev165399, Aug. 2018]. To obtain specific differentiated organ cells, differentiation steps that take place in normal embryonic development have to be recaptured, either completely in vitro, or alternatively in part in vivo after delivery of progenitor cells to the patient.

Using stem cells, especially iPS cells, to generate cell types for tissue repair holds great clinical potential for treating and potentially curing a multifold of diseases. However to enable real regenerative medicine based on stem cell therapy, many hurdles still need to be taken, in part due to still incomplete understanding of how to generate the right cells with sufficient purity [Badylak et al., npj Regenerative Medicine, vol. 2, no. 1, p. 2, Jan. 2017]. Therefore there is a need for assays to ascertain that various used batches of stem cells have the same characteristics, to define the differentiation steps towards the cell types required for mimicking the disease, and to quantify the purity and differentiation status of the cell batch to be returned to the patient after in vitro culture and manipulation.

A problem with stem cell lines, including iPS lines, do not always have the same pluripotency characteristics. This in part depends on the way they have been generated [Strano et al., https://doi.org/10.1016/j.celrep.2020.107732]. Establishing and maintaining the pluripotency state of a human stem cell line in culture is a challenge [International Stem Cell Initiative, Nat Commun, vol. 9, no. 1, p. 1925, 15 2018]. Multiple signaling pathways orchestrate the state of stem cell pluripotency, e.g. the PI3K, Wnt, Hedgehog, TGFβ, STAT3 pathways [Ying et al., Stem Cell Reports, vol. 8, no. 6, pp. 1457-1464, Jun. 2017; Sokol, Development, vol. 138, no. 20, pp. 4341-4350, Oct. 2011; Huang et al., Cell Res., vol. 19, no. 10, pp. 1127-1138, Oct. 2009; Massagué, Nat. Rev. Mol. Cell Biol., vol. 13, no. 10, pp. 616-630, Oct. 2012; Yu et al., Development, vol. 143, no. 17, pp. 3050-3060, Sep. 2016]. Therefore there is a high need for assays that quantify the degree of pluripotency, and differentiation capability into the various differentiation lineages, and also enables quantitative comparison of various used batches of stem cells, e.g. before and after freezing, after passaging the stem cell culture to avoid selection for a stem cell clone(s) with different characteristics.

Development of iPS derived disease models comes with challenges, the most important being reproducibility in development of the disease model [van de Stolpe et al., Lab Chip, vol. 13, no. 18, pp. 3449-3470, Sep. 2013]. This requires standardized differentiation steps to reliably obtain the required cell or tissue type. Therefore there is a high need for assays that quantify the degree of pluripotency, and differentiation capability into the various differentiation lineages, an also enables quantitative comparison of various used batches of (differentiated) stem cells.

Specific challenges in the use of stem cells for clinical regenerative medicine

To obtain regulatory approval for clinical use, the cell culture and differentiation should be very controlled and reproducible, requiring strict quality control protocols and standardization. With clinical use of stem cell derivatives for regenerative medicine purposes come a large number of quality control requirements. We anticipate that sequential differentiation steps, e.g. passing through a definitive endoderm stage to obtain insulin-producing pancreatic cells, will need to be documented, as well as a detailed characterization of the end-product. Signaling pathway activity analysis may enable a thorough and quantitative biological assessment of stem cell derivatives, as well as comparison between different batches of cells that re presumed to have the same differentiation characteristics [Dimmeler et al., Nat. Med., vol. 20, no. 8, pp. 814-821, Aug. 2014]. Therefore there is a high need for assays that enables quantitative comparison of various used batches of stem cells, and to quantify a purity of a certain desired cell type in a cell culture.

Stem cell lines, especially iPS cell lines, are important in generating disease models and form the backbone of regenerative medicine, both in development of therapies as well as in clinical use. However, they differ with respect to phenotype (RNA and/or protein expression) and importantly, differentiation capabilities. These are the result of different underlying genetics, even when iPS cell line generation and culture protocols are standardized.

This presents a huge problem for experimental reproducibility with using these cell lines or their differentiated derivatives for all kinds of purposes, and especially for development of regenerative medicine therapies as well as when administering cell-based therapies to the patient. Therefore standardized quality control assays are very needed to quantify the pluripotency or multipotency or progenitor state, as well as the differentiation potential, and the obtained differentiation state during research or cell batch preparation for cell therapy.

The above needs are met and the above problems are overcome by the methods and products as defined in the appended claims.

### SUMMARY OF THE INVENTION

To further the field of stem cell technology, methods are needed to specify in a quantitative manner the pluripotency and differentiation state of stem cells. Therefore, in a first aspect of the invention is provided an *in vitro* or *ex vivo* method for determining the differentiation state of a stem cell, based on determining, or the result of a determining of the activities of at least three cellular signaling pathways selected from the groups consisting of TGFbeta, Notch, JAK-STAT3, Hedgehog, and Wnt, the method comprising:
- comparing the at least three cellular signaling pathway activities in the stem cell with at least one reference cellular signaling pathway activity;
- determining the differentiation state of the stem cell based on the compared cellular signaling pathway activities, wherein the differentiation state of the stem cell is determined to be pluripotent, multipotent, unipotent, or at least partially differentiated.

It is important to quantitatively characterize stem cell lines, as well as different batches or passages of the same cell line, with respect to signaling pathway activity and accurately define a functional state of pluripotency for each cell line or passage of a cell line. Assays are needed that can measure activity of these pathways simultaneously in a cell or tissue sample, at various stages in the differentiation process [Yu et al., Development, vol. 143, no. 17, pp. 3050-3060, Sep. 2016]. Similar issues play in characterization of multipotent and progenitor stem cells.

The method disclosed herein provides such quantitative characterization of stem cells by determining the activities of multiple cellular signaling pathways. As demonstrated in the examples provided below, doing so allows determining the pluripotency of the analyzed stem cells or determine whether these stem cells are (partially) differentiated. Single assays that are able to quantitatively determine the pluripotency or differentiation state of a stem cell sample have not been described in the art and are a useful tool to further the field of stem cell research and stem cell medicine.

Using a calibrated mathematical to determine the cellular signaling pathway activity (e.g. based on the expression levels of target genes) allows the comparison between different samples. The mathematical model provides a numerical value for each determined pathway activity, which can be compared with the values obtained in one or more other samples. Thereby rendering it possible to use the method for different purposes, such as quality control of a stem cell sample by comparing the cellular signaling pathway activities with desired values (e.g. obtained in a validated reference sample or samples). The method can also be used to determine the differentiation state by comparing the pathway activities with pathway activities obtained from e.g. a pluripotent stem cell and pathway activities obtained from different stages of differentiation of said or similar stem cells.

By analyzing the determined pathway activities it can be determined whether these activities match the expected pathway activities for the type of stem cell being analyzed. For example the signaling pathway activities for an iPS cell line are well defined and also indicated in the results below, allowing comparison of signaling pathway activities determined in an iPS sample with expected or reference values. As for example further demonstrated in the examples below, hES cells may be cultured in different conditions, e.g. with or without feeder layer, further a human cell or a mouse cell feeder layer may be used, or when not using a feeder layer conditioned medium form either human or mouse cells, or in a defined medium with known constituents which can be varied on demand. It is known in the art that only hES cells grown on a feeder layer of human cells display true pluripotent properties, meaning these stem cells are capable of forming each of the three germ layers. Other culture conditions that are easier to use, have been investigated and defined trying to copy this pluripotency state. In the examples it is demonstrated that a clear distinction can be made between cells grown in these different conditions. E.g. true pluripotent hES cells grown on a T3HDF (human cell) feeder layer display low FOXO and Wnt signaling activity, intermediate TGFbeta signaling activity and high Hedgehog, Notch and STAT3 signaling activities. hES cells grown in T3HDF cultured medium show similar properties but differ in that they display intermediate FOXO signaling activity, and STAT3 signaling activity appears somewhat decreased. When hES are grown on mouse feeder layers or cultured medium, they display low TGFbeta signaling and intermediate Notch and STAT3 signaling activities.

These differences in signaling pathway activity may account for their reduced ability to differentiate in each germ layer and cells derived from it. However it also demonstrates the ability of using signaling pathway activities to characterize stem cells, or use pathway activities as a quality control tool. In other words, by analyzing the pathway activities of a stem cell sample, e.g. a hES or iPS sample, a prediction can be made regarding the ability of the stem cells to be able to differentiate to each of the three germ layers (its pluripotency), and hence the quality of the stem cell sample.

The method disclosed herein is preferably performed on cultured stem cells, but may also be performed on stem cells extracted from or present in a sample obtained from a subject, such as a human or non-human animal.

When used herein, differentiation state refers to the ability of the stem cell to differentiate into the different lineages or cell types such stem cell is *in vivo* is capable of differentiating into. This may be different depending on the type of stem cell. For example, an hES or iPS (both pluripotent stem cells) can in principle differentiate into each of the three germ layers and subsequently can give rise any cell type, thus their differentiation state is determined as their ability to form each of the three germ layers and is reflected in their pluripotency. On the other hand an organ stem cell can either self-renew or differentiate into a specific subtype of specialized cells, therefore its differentiation state is reflected by its ability to self-renew and its multipotency, i.e. its ability to form specialized cells present in the respective organ it is derived from.

Therefore the differentiation state of a stem cell can be determined by comparison of the determined signaling pathway activities of said stem cell with the expected signaling pathway activities for said particular stem cell, e.g. values obtained in a reference sample or derived from general knowledge in the field.

When used herein, determining an activity of a cellular signaling pathway refers to any method capable of determining and quantifying the activity level of a signaling pathway. For example the methods described herein below may be used, by determining the expression level of at least three target genes of each of the cellular signaling pathways and correlating the expression levels of these target genes to the activity of the cellular signaling pathways. However other methods are known to the skilled person that may be used to determine pathway activity, such methods not limited to immunohistochemistry, Western blotting, Northern blotting, localization assays, phosphorylation specific antibodies, and the like.

Preferably the method is based on determining or the result of a determining of the activities of three or more cellular signaling pathways, such as three, four, five, six, seven, eight, nine, ten or more cellular signaling pathways. Although it is recognized that determining as many cellular signaling pathway activities as possible would provide the most information, it was found that a minimum of three cellular signaling pathways selected from TGFbeta, Notch, JAK-STAT3, Hedgehog, and Wnt suffice for all analyzed stem cells types to distinguish between its pluripotent (or multipotent) state and partially differentiated states. Therefore in a more preferred embodiment the method is based on determining or the result of a determining of the activities of three or more cellular signaling pathways, such as three, four, five, six, seven, eight, nine, ten or more cellular signaling pathways comprising three or more cellular signaling pathways, e.g. three, four or five, selected form the group consisting of TGFbeta, Notch, JAK-STAT3, Hedgehog, and Wnt.

When used herein, the step of comparing the at least three cellular signaling pathway activities in the stem cell with at least one reference cellular signaling pathway activity, refers to step where the determined pathway activities are compared with an internal or external reference comprising at least one pathway activity. The at least one reference cellular signaling pathway may be determined on the stem cell sample itself, e.g. simultaneous with the three or more cellular signaling pathways. In such case the reference cellular signaling pathway may serve as an internal control to normalize the determined three or more pathway activities. In such case preferably the step further comprises assessing the determined pathway activities based on expected pathway activities, wherein the assessing comprising determining for each pathway activity whether it is as expected, lower than expected or higher than expected.

For example in hES Wnt signaling is very low and relatively constant. Therefore the Wnt cellular signaling activity can be used as an internal control to normalize the active pathways such as Hedgehog, Notch and STAT3. This can for example be done by calculating relative activities or ratios of the HH, Notch and STAT3 pathways with respect to Wnt pathway. Alternatively the three or more cellular activities can be compared with pathway activities in a reference sample, for example the same three or more cellular signaling pathways activities.

Alternatively and more preferably the reference cellular signaling pathway may be determined in a reference sample, preferably a reference sample of a similar or identical type stem cell type. In such case the reference cellular signaling pathway activity or activities may be used in a direct comparison with the determined three or more pathway activities. It is understood that the two options may also be combined, meaning an internal control may be used to normalize the determined three or more pathway activities, and subsequently the normalized three or more pathway activities may be compared with reference pathways determined in a control sample or stem cell.

Therefore in a preferred embodiment, the method comprises:
- comparing the at least three cellular signaling pathway activities in the stem cell with at least three or more reference cellular signaling pathway activity;
Therefore in a more preferred embodiment, the method comprises:
- comparing the at least three cellular signaling pathway activities in the stem cell with at least three or more reference cellular signaling pathway activity, wherein the three or more cellular signaling pathways and the three or more reference cellular signaling pathways are the same pathways;

For example, HH, Notch and STAT3 signaling pathway activities may be determined in a validated reference hES culture to obtain reference pathway activities, or a range of pathway activities. In a stem cell sample to be determined the Notch and STAT3 signaling pathway activities can be determined and directly compared with the determined reference pathway activities.

It is understood that multiple references may be used in the comparing step. In such case the average numeric values for each cellular signaling pathway can be determined among the multiple reference samples and the comparison step can be performed with the determined average value. Doing so provides the further advantage that a standard deviation can be determined which can be used as a threshold for determining whether a pathway activity is higher or lower as described herein. Alternatively the different values for each pathway activity determined in multiple references may be used to specify a range of expected values, for the specific conditions of the reference samples.

By using a calibrated mathematical model to relate the gene expression levels to a cellular signaling pathway activity, a numerical value can be assigned to the pathway activity. Depending on the model, this value can for example be normalized to result in a value from 0 to 100, where 0 is no pathway activity and 100 is the theoretical maximum pathway activity. Alternatively the value may be normalized such that the average value is 0 and thus decreased pathway activity is represented by a negative value and increased pathway activity is represented by a positive value. It is understood that the values obtained using such model are dependent on the model used, and do not represent absolute values. Therefore, the same model should be used for calibrating, determining reference values and when used in the method of the invention, so that it allows comparison of the obtained numerical values for pathway activities.

Therefore, the numerical value obtained for a pathway activity in a sample may be compared to the numerical value obtained for that pathway activity in a reference sample. By performing such comparison a statement can be made about the pathway activity in the sample (e.g. a stem cell sample) relative to the pathway activity determined in the reference sample (e.g. a stem cell sample). Based on the numerical value a statement can be made about the pathway activity in the sample with respect to the pathway activity in the reference sample, e.g. whether the pathway activity in the sample is higher or lower, in correspondence with the numerical value obtained for the pathway activity. E.g. the Wnt cellular signaling pathway activity can be determined in a reference stem cell sample, e.g. a reference hES sample. Since it is determined by the inventors that the Wnt cellular signaling pathway activity in a hES is very low, this can be set as a baseline numerical value representing an inactive pathway, i.e. the reference value. By determining the numerical value for the Wnt cellular signaling pathway activity in the hES sample to be analyzed, the numerical value representing that pathway activity can be compared with the reference value, and it can be determined whether the pathway activity is higher or equal (or lower) compared to the reference based on the numerical values. The comparison may be made with multiple reference samples to allow for more accurate results and statistics to be performed. Alternatively a reference sample can be used to set a baseline pathway activity, allowing further comparison of the pathway activity. For example, the average and standard deviation can be calculated which can be used to calculate values for a pluripotent, multipotent, or unipotent stem cell, and define a threshold for abnormal pathway activity.

Therefore, in a preferred embodiment the pathway activity is determined to be higher or lower when the obtained numerical value for the pathway activity differs with at least one standard deviation from the numerical value obtained for the pathway activity in the reference sample. When the value is within the range of one standard it is said to be equal or comparable to the pathway activity in the reference sample, and consequently when it is one or more standard deviation higher the activity said to be "high" or "higher". It is understood that the threshold may also be set higher, for example 2 times the standard deviation or even 3 times the standard deviation. It is further understood that the threshold may be determined using alternative ways, e.g. statistical methods, to determine a significant deviation from the reference value. Alternatively a preset value can be used a cutoff for the activity of a pathway to be considered abnormal (with reference to the reference sample pathway activity).

It is understood that the reference value for a certain pathway activity in a sample in principle only needs to be determined once. Therefore, the step of determining a reference pathway activity is not part of the methods of the invention, as a predetermined reference pathway activity can be used.

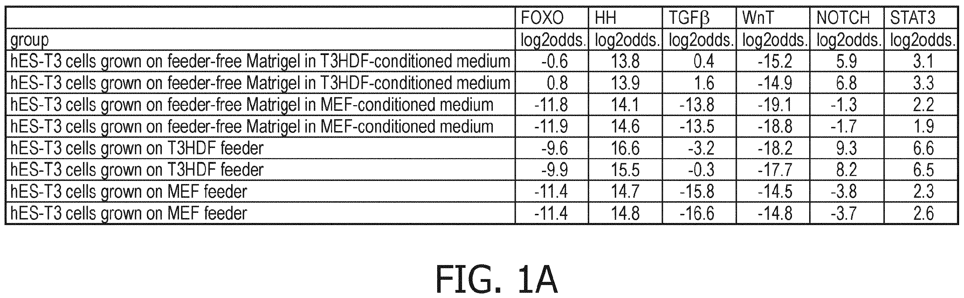

When referring to figure 1A and the examples provided below the FOXO, Hedgehog, TGFbeta, Wnt, Notch and STAT3 cellular signaling pathway activities were determined for different stem cell samples. Human embryonic stem cells when grown in culture can be truly pluripotent when grown in the right culturing conditions. It is known that the cell can be grown with or without feeder layer to supply the cells with the required growth factors. When grown in the absence of a feeder layer the cells need to be cultured in medium obtained from the feeder layer cells. The feeder layer can be human or mouse cells. It is known in the art that using a feeder layer of human cells results in truly pluripotent results, and that cells grown in different conditions still have capacity for differentiation but shows differences in properties from cells grown in this way. The data presented in Fig. 1A demonstrates that the differences between these culturing conditions can be seen at the signaling pathway level. Although some of these signaling pathways are known to be important in maintaining stem cell pluripotency or differentiation, this is the first time that a plurality of signaling pathway activities can be analyzed in a quantitative and calibrated manner suitable for comparing different samples among each other.

This is useful for several reasons, as these data provide a reference for a set of signaling pathway activities and the pathway activities of a test stem cells sample may be compared with these activities to determine whether the test sample has the desired signaling pathway activities (e.g. those displayed by the ES grown on a human feeder layer).

For example Fig. IB displays signaling pathway activities of several iPS and hES samples. From this figure it can be concluded that although comparable, the iPS samples deviate in pathway activities for example in Notch and TGFbeta signaling when compared to the ES samples. These differences may be attributed to different cell characteristics (e.g. pluripotency or differentiation) or culturing conditions. Further these results can be compared with the results from Fig. 1A demonstrating that the H7 ES sample displays pathway activities most in line with hES grown on a human feeder layer thus likely representing a pluripotent stem cell sample.

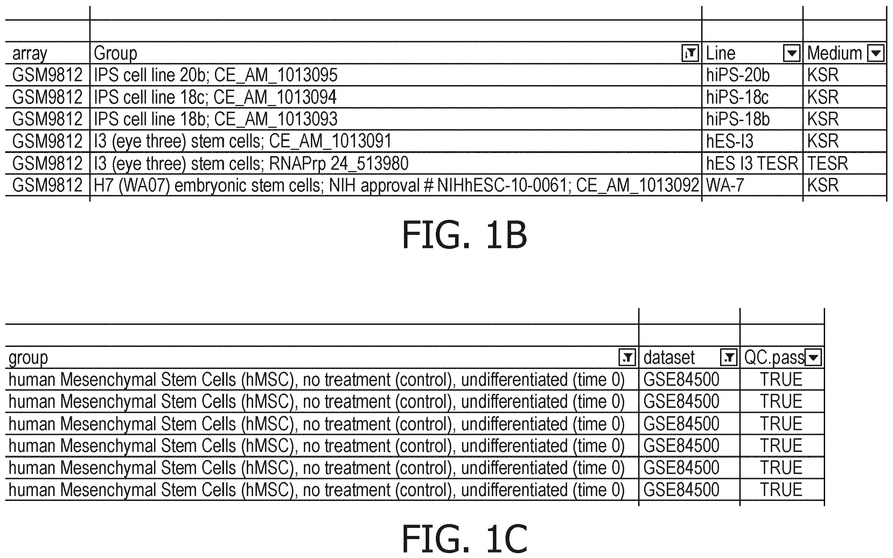

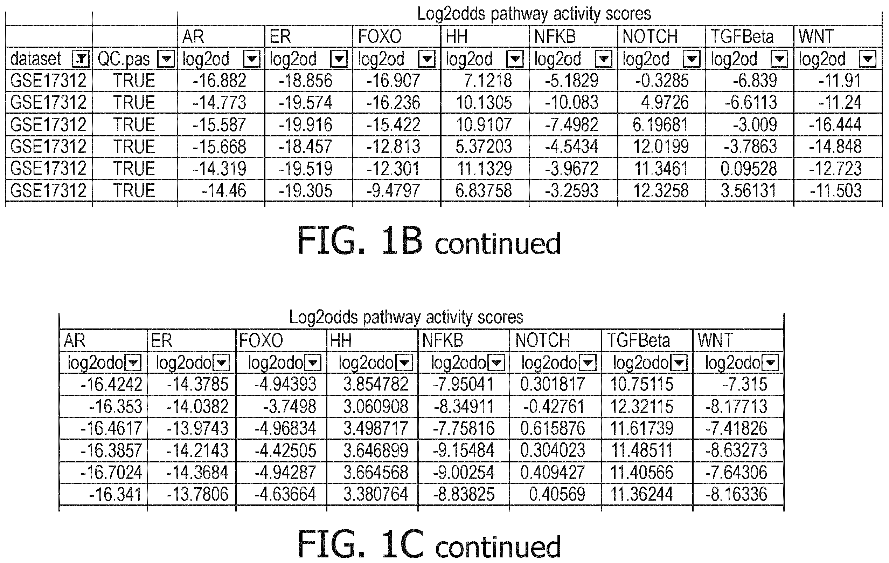

Fig. 1C displays pathway activities for mesenchymal stem cells. These stem cells are multipotent and of mesodermal origin. The pathway activities can be clearly distinguished from pluripotent stem cells as the mesenchymal stem cells display higher TGFbeta activity and lower Notch activity.

For the purpose of the invention determining the expression levels of the target genes may be based on extracted RNA. The steps of determining the expression levels and/or extracting RNA from the sample may be a part of the method, meaning the method may include the step of determining the expression levels of the target genes on RNA extracted from the stem cell sample using methods known to the skilled person or described herein. Alternatively the expression levels may have been determined separately and the determining step (of the expression levels of the target genes) is not an active step in the method of the invention. In such case the expression levels are provided as an input value, e.g. a relative expression level in reference to one or more control gene expression levels.

When used herein, "expression level" refers to quantifying the number of mRNA copies transcribed from a gene. Generally this number will not be an absolute value but a relative value, and therefore is preferably normalized for example in reference to the expression of one or more housekeeping genes. Housekeeping genes are genes which are assumed to have constant expression levels independent of cell type and/or functional status of the cell (i.e. from a diseased or healthy subject), and therefore can be used to normalize experimentally determined relative expression levels. Housekeeping genes are generally known to the skilled person, non-limiting examples of housekeeping genes that may be used for normalization are beta-actin, glyceraldehyde-3-phosphate dehydrogenase (GAPDH) and Transcription factor IID TATA binding protein (TBP).

Therefore the phrase "expression level of a target gene" denotes a value that is representative of the amount, e.g. a concentration, of a target gene present in a sample. This value may be based on the amount of a transcription product of the target gene (e.g. mRNA) or a translation product thereof (e.g. protein). Preferably, the expression level is based on the amount of mRNA formed from the target gene. In order to determine the expression level, techniques such as qPCR, multiple qPCR, multiplexed qPCR, ddPCR, RNAseq, RNA expression array or mass spectrometry may be used. For example, a gene expression microarray, e.g. Affymetrix microarray, or RNA sequencing methods, like an Illumina sequencer, can be used.

Sets of cellular signaling pathway target genes whose expression levels are preferably analyzed have been identified, alternatively methods for identifying suitable target genes are described herein. For use to determine pathway activity, for example by a mathematical model, three or more, for example, three, four, five, six, seven, eight, nine, ten, eleven, twelve or more, target genes from each assessed cellular signaling pathway can be analyzed to determine pathway activities.

In an embodiment the signaling pathway measurements are performed using qPCR, multiple qPCR, multiplexed qPCR, ddPCR, RNAseq, RNA expression array or mass spectrometry. For example, a gene expression microarray data, e.g. Affymetrix microarray, or RNA sequencing methods, like an Illumina sequencer, can be used.

The term "subject", as used herein refers to any living being. In some embodiments, the subject is an animal, preferably a mammal. In certain embodiments, the subject is a human being, such as a medical subject. Although the invention is not necessarily limited to a particular group of subjects, it will be apparent that a subject having infection or a subject suspected to have an infection or a subject at risk for developing infection profits the most form the invention described herein. The method is particularly useful for a subject having a viral infection.

The terms "pathway", "signal transduction pathway", "signaling pathway" and "cellular signaling pathway" are used interchangeably herein.

An "activity of a signaling pathway" may refer to the activity of a signaling pathway associated transcription factor (TF) element in the sample, the TF element controlling transcription of target genes, in driving the target genes to expression, i.e., the speed by which the target genes are transcribed, e.g. in terms of high activity (i.e. high speed) or low activity (i.e. low speed), or other dimensions, such as levels, values or the like related to such activity (e.g. speed). Accordingly, for the purposes of the present invention, the term "activity", as used herein, is also meant to refer to an activity level that may be obtained as an intermediate result during "pathway analysis" as described herein.

The term "transcription factor element" (TF element), as used herein, preferably refers to an intermediate or precursor protein or protein complex of the active transcription factor, or an active transcription factor protein or protein complex which controls the specified target gene expression. For example, the protein complex may contain at least the intracellular domain of one of the respective signaling pathway proteins, with one or more co-factors, thereby controlling transcription of target genes. Preferably, the term refers to either a protein or protein complex transcriptional factor triggered by the cleavage of one of the respective signaling pathway proteins resulting in a intracellular domain.

The term "target gene", as used herein, means a gene whose transcription is directly or indirectly controlled by a respective transcription factor element. The "target gene" may be a "direct target gene" and/or an "indirect target gene" (as described herein).

Pathway analysis enables quantitative measurement of signal transduction pathway activity in blood cells, based on inferring activity of a signal transduction pathway from measurements of mRNA levels of the well-validated direct target genes of the transcription factor associated with the respective signaling pathway (see for example W Verhaegh, A van de Stolpe, Oncotarget, 2014, 5(14):5196).

Preferably the determining of the activity or activities of the signaling pathway(s), the combination of multiple pathway activities and applications thereof is performed as described for example in the following documents, each of which is hereby incorporated in its entirety for the purposes of determining activity of the respective signaling pathway: published international patent applications WO2013011479 (titled "ASSESSMENT OF CELLULAR SIGNALING PATHWAY ACTIVITY USING PROBABILISTIC MODELING OF TARGET GENE EXPRESSION"), WO2014102668 (titled "ASSESSMENT OF CELLULAR SIGNALING PATHWAY ACTIVITY USING LINEAR COMBINATION(S) OF TARGET GENE EXPRESSIONS"), WO2015101635 (titled "ASSESSMENT OF THE PI3K CELLULAR SIGNALING PATHWAY ACTIVITY USING MATHEMATICAL MODELLING OF TARGET GENE EXPRESSION"), WO2016062891 (titled "ASSESSMENT OF TGF-β CELLULAR SIGNALING PATHWAY ACTIVITY USING MATHEMATICAL MODELLING OF TARGET GENE EXPRESSION"), WO2017029215 (titled "ASSESSMENT OF NFKB CELLULAR SIGNALING PATHWAY ACTIVITY USING MATHEMATICAL MODELLING OF TARGET GENE EXPRESSION"), WO2014174003 (titled "MEDICAL PROGNOSIS AND PREDICTION OF TREATMENT RESPONSE USING MULTIPLE CELLULAR SIGNALLING PATHWAY ACTIVITIES"), WO2016062892 (titled "MEDICAL PROGNOSIS AND PREDICTION OF TREATMENT RESPONSE USING MULTIPLE CELLULAR SIGNALING PATHWAY ACTIVITIES"), WO2016062893 (titled "MEDICAL PROGNOSIS AND PREDICTION OF TREATMENT RESPONSE USING MULTIPLE CELLULAR SIGNALING PATHWAY ACTIVITIES"), WO2018096076 (titled "Method to distinguish tumor suppressive FOXO activity from oxidative stress"), and in the patent applications WO2018096076 (titled "Method to distinguish tumor suppressive FOXO activity from oxidative stress"), WO2019068585 (titled "Assessment of Notch cellular signaling pathway activity using mathematical modelling of target gene expression"), WO2019120658 (titled "Assessment of MAPK-MAPK-AP1 cellular signaling pathway activity using mathematical modelling of target gene expression"), WO2019068543 (titled "Assessment of JAK-JAK-STAT3 cellular signaling pathway activity using mathematical modelling of target gene expression"), WO2019068562 (titled "Assessment of JAK-STAT1/2 cellular signaling pathway activity using mathematical modelling of target gene expression"), and WO2019068623 (titled "Determining functional status of immune cells types and immune response").

The models have been biologically validated for ER, AR, PI3K-FOXO, HH, Notch, TGF-β, Wnt, NFkB, JAK-STAT1/2, JAK-JAK-STAT3 and MAPK-MAPK-AP1 pathways on several cell types.

Unique sets of cellular signaling pathway target genes whose expression levels are preferably analyzed have been identified. For use in the mathematical models, three or more, for example, three, four, five, six, seven, eight, nine, ten, eleven, twelve or more, target genes from each assessed cellular signaling pathway can be analyzed to determine pathway activities.

Common to the pathway analysis methods for determining the activities of the different signaling pathways as disclosed herein is a concept, which is preferably applied herein for the purposes of the present invention, wherein the activity of a signaling pathway in a cell such as a cell present in a blood sample is determinable by receiving expression levels of one or more, preferably three or more, target genes of the signaling pathway, determining an activity level of a signaling pathway associated transcription factor (TF) element in the sample, the TF element controlling transcription of the three or more target genes, the determining being based on evaluating a calibrated mathematical pathway model relating expression levels of the one or more, preferably three or more target genes to the activity level of the signaling pathway, and optionally inferring the activity of the signaling pathway in the cell present in a blood sample based on the determined activity level of the signaling pathway associated TF element. As described herein, the activity level can be directly used as an input to determine the immune response in a subject and/or determine whether an infection is viral and/or determine the severity of a viral infection and/or determine the cellular immmunity conferred by a vaccine, which is also contemplated by the present invention.

The term "activity level" of a TF element, as used herein, denotes the level of activity of the TF element regarding transcription of its target genes.

The calibrated mathematical pathway model may be a probabilistic model, preferably a Bayesian network model, based on conditional probabilities relating the activity level of the signaling pathway associated TF element and the expression levels of the three or more target genes, or the calibrated mathematical pathway model may be based on one or more linear combination(s) of the expression levels of the three or more target genes. For the purposes of the present invention, the calibrated mathematical pathway model is preferably a centroid or a linear model, or a Bayesian network model based on conditional probabilities.

In particular, the determination of the expression level and optionally the inferring of the activity of a signaling pathway in the subject may be performed, for example, by inter alia (i) evaluating a portion of a calibrated probabilistic pathway model, preferably a Bayesian network, representing the cellular signaling pathways for a set of inputs including the expression levels of the three or more target genes of the cellular signaling pathway measured in a sample of the subject, (ii) estimating an activity level in the subject of a signaling pathway associated transcription factor (TF) element, the signaling pathway associated TF element controlling transcription of the three or more target genes of the cellular signaling pathway, the estimating being based on conditional probabilities relating the activity level of the signaling pathway associated TF element and the expression levels of the three or more target genes of the cellular signaling pathway measured in the sample of the subject, and optionally (iii) inferring the activity of the cellular signaling pathway based on the estimated activity level of the signaling pathway associated TF element in the sample of the subject. This is described in detail in the published international patent application WO 2013/011479 A2 ("Assessment of cellular signaling pathway activity using probabilistic modeling of target gene expression"), the contents of which are herewith incorporated in their entirety.

In an exemplary alternative, the determination of the expression level and optionally the inferring of the activity of a cellular signaling pathway in the subject may be performed by inter alia (i) determining an activity level of a signaling pathway associated transcription factor (TF) element in the sample of the subject, the signaling pathway associated TF element controlling transcription of the three or more target genes of the cellular signaling pathway, the determining being based on evaluating a calibrated mathematical pathway model relating expression levels of the three or more target genes of the cellular signaling pathway to the activity level of the signaling pathway associated TF element, the mathematical pathway model being based on one or more linear combination(s) of expression levels of the three or more target genes, and optionally (ii) inferring the activity of the cellular signaling pathway in the subject based on the determined activity level of the signaling pathway associated TF element in the sample of the subject. This is described in detail in the published international patent application WO 2014/102668 A2 ("Assessment of cellular signaling pathway activity using linear combination(s) of target gene expressions"), the contents of which are herewith incorporated in their entirety.

Further details regarding the inferring of cellular signaling pathway activity using mathematical modeling of target gene expression can be found in W Verhaegh et al., "Selection of personalized patient therapy through the use of knowledge-based computational models that identify tumor-driving signal transduction pathways", Cancer Research, Vol. 74, No. 11, 2014, pages 2936 to 2945.

To facilitate rapid identification of references, the above-mentioned references have been assigned to each signaling pathway of interest here and exemplarily corresponding target genes suitable for determination of the signaling pathway's activity have been indicated. In this respect, particular reference is also made to the sequence listings for the target genes provided with the above-mentioned references.
AR: KLK2, PMEPA1, TMPRSS2, NKX3 1, ABCC4, KLK3, FKBP5, ELL2, UGT2B15, DHCR24, PPAP2A, NDRG1, LRIG1, CREB3L4, LCP1, GUCY1A3, AR, EAF2, APP, NTS, PLAU, CDKN1A, DRG1, FGF8, IGF1, PRKACB, PTPN1, SGK1 and TACC2 (WO 2013/011479, WO 2014/102668); KLK2, PMEPA1, TMPRSS2, NKX3 1, ABCC4, KLK3, FKBP5, ELL2, UGT2B15, DHCR24, PPAP2A, NDRG1, LRIG1, CREB3L4, LCP1, GUCY1A3, AR, and EAF2 (WO 2014/174003);
ER: CDH26, SGK3, PGR, GREB1, CA12, XBP1, CELSR2, WISP2, DSCAM, ERBB2, CTSD, TFF1, PDZK1, IGFBP4, ESR1, SOD1, AP1B1, NRIP1, AP1B1, ATP5J, COL18A1, COX7A2L, EBAG9, ESR1, HSPB1, IGFBP4, KRT19, MYC, NDUFV3, PISD, PRDM15, PTMA, RARA, SOD1 and TRIM25 (WO 2013/011479, WO 2014/174003);
WNT: KIAA1199, AXIN2, RNF43, TBX3, TDGF1, SOX9, ASCL2, IL8, SP5, ZNRF3, KLF6, CCND1, DEFA6, FZD, NKD1, OAT, FAT1, LEF1, GLUL, REG1B, TCF7L2, COL18A1, BMP7, SLC1A2, ADRA2C, PPARG, DKK1, HNF1A and LECT2 (WO 2014/174003);
HH: GLI1, PTCH1, PTCH2, IGFBP6, SPP1, CCND2, FST, FOXL1, CFLAR, TSC22D1, RAB34, S100A9, S100A7, MYCN, FOXM1, GLI3, TCEA2, FYN, CTSL1, BCL2, FOXA2, FOXF1, H19, HHIP, IL1R2, JAG2, JUP, MIF, MYLK, NKX2.2, NKX2.8, PITRM1 and TOM1 (WO 2014/174003);
NOTCH: CD28, CD44, DLGAP5, DTX1, EPHB3, FABP7, GFAP, GIMAP5, HES1, HES4, HES5, HES7, HEY1, HEY2, HEYL, KLF5, MYC, NFKB2, NOX1, NRARP, PBX1, PIN1, PLXND1, PTCRA, SOX9, and TNC (WO 2019/068585 A1);
PI3K: AGRP, BCL2L11, BCL6, BNIP3, BTG1, CAT, CAV1, CCND1, CCND2, CCNG2, CDK 1A, CDK 1B, ESR1, FASLG, FBX032, GADD45A, INSR, MXIl, NOS3, PCK1, POMC, PPARGCIA, PRDX3, RBL2, SOD2, TNFSF10, ATP8A1, C100rf10, CBLB, DDB1, DYRK2, ERBB3, EREG, EXT1, FGFR2, IGF1R, IGFBP1, IGFBP3, LGMN, PPM ID, SEMA3C, SEPP1, SESN1, SLC5A3, SMAD4, TLE4, ATG14, BIRC5, IGFBP1, KLF2, KLF4, MYOD1, PDK4, RAG1, RAG2, SESN1, SIRT1, STK11 and TXNIP (WO 2015/101635 A1); SOD2, BNIP3, MXIl, PCKl, PPARGCIA and CAT (WO 2018/096076 A1)
JAK-STAT1/2: BID, GNAZ, IRF1, IRF7, IRF8, IRF9, LGALS1, NCF4, NFAM1, OAS1, PDCD1, RAB36, RBX1, RFPL3, SAMM50, SMARCB1, SSTR3, ST13, STAT1, TRMT1, UFD1L, USP18, and ZNRF3, preferably, from the group consisting of: IRF1, IRF7, IRF8, IRF9, OAS1, PDCD1, ST13, STAT1 and USP18 (WO 2019/068562A1);
JAK-STAT3: AKT1, BCL2, BCL2L1, BIRC5, CCND1, CD274, CDKN1A, CRP, FGF2, FOS, FSCN1, FSCN2, FSCN3, HIF1A, HSP90AA1, HSP90AB1, HSP90B1, HSPA1A, HSPA1B, ICAM1, IFNG, IL10, JunB, MCL1, MMP1, MMP3, MMP9, MUC1, MYC, NOS2, POU2F1, PTGS2, SAA1, STAT1, TIMP1, TNFRSF1B, TWIST1, VIM and ZEB1 (WO 2019/068543 A1);
MAPK-AP-1: BCL2L11, CCND1, DDIT3, DNMT1, EGFR, ENPP2, EZR, FASLG, FIGF, GLRX, IL2, IVL, LOR, MMP1, MMP3, MMP9, SERPINE1, PLAU, PLAUR, PTGS2, SNCG, TIMP1, TP53 and VIM (WO 2019/120658 A1);
NFkB: BCL2L1, BIRC3, CCL2, CCL3, CCL4, CCL5, CCL20, CCL22, CX3CL1, CXCL1, CXCL2, CXCL3, ICAM1, IL1B, IL6, IL8, IRF1, MMP9, NFKB2, NFKBIA, NFKB IE, PTGS2, SELE, STAT5A, TNF, TNFAIP2, TNIP1, TRAF1 and VCAM1 (WO 2017/029215); TGFbeta: ANGPTL4, CDC42EP3, CDKNIA, CDKN2B, CTGF, GADD45A, GADD45B, HMGA2, ID1, IL11, SERPINE1, INPP5D, JUNB, MMP2, MMP9, NKX2-5, OVOL1, PDGFB, PTHLH, SGK1, SKIL, SMAD4, SMAD5, SMAD6, SMAD7, SNAI1, SNAI2, TIMP1 and VEGFA (WO 2016/062891, WO 2016/062893);

Particularly preferred is a method wherein the inferring comprises:
inferring activity of a Wnt cellular signaling pathway in the sample based at least on expression levels of three or more, e.g. three, four five six seven, eight, nine, ten eleven, twelve or thirteen or more, target gene(s) of the Wnt pathway measured in the sample selected from the group comprising or consisting of: KIAA1199, AXIN2, RNF43, TBX3, TDGF1, SOX9, ASCL2, IL8, SP5, ZNRF3, KLF6, CCND1, DEFA6 and FZD7, optionally the inferring is further based on expression levels of at least one target gene, e.g. one, two three, four, five six, seven, eight, nine, ten or more target gene(s), of the Wnt pathway measured in the sample selected from the group comprising or consisting of: NKD1, OAT, FAT1, LEF1, GLUL, REG1B, TCF7L2, COL18A1, BMP7, SLC1A2, ADRA2C, PPARG, DKK1, HNF1A and LECT2;
inferring activity of a ER cellular signaling pathway in the sample based at least on expression levels of three or more, e.g. three, four five six seven, eight, nine, ten eleven, twelve or thirteen, target gene(s) of the ER pathway measured in the sample selected from the group comprising or consisting of: CDH26, SGK3, PGR, GREB1, CA12, XBP1, CELSR2, WISP2, DSCAM, ERBB2, CTSD, TFF1 and NRIP1, optionally the inferring is further based on expression levels of at least one target gene, e.g. one, two, three, four, five, six, seven, eight, nine, ten eleven twelve or more target gene(s), of the ER pathway measured in the sample selected from the group comprising or consisting of: AP1B1, ATP5J, COL18A1, COX7A2L, EBAG9, ESR1, HSPB1, IGFBP4, KRT19, MYC, NDUFV3, PISD, PRDM15, PTMA, RARA, SOD1 and TRIM25;
inferring activity of a HH cellular signaling pathway in the sample based at least on expression levels of three or more, e.g. three, four five six seven, eight, nine, ten eleven, twelve or thirteen, target gene(s) of the HH pathway measured in the sample selected from the group comprising or consisting of: GLI1, PTCH1, PTCH2, IGFBP6, SPP1, CCND2, FST, FOXL1, CFLAR, TSC22D1, RAB34, S100A9, S100A7, MYCN, FOXM1, GLI3, TCEA2, FYN and CTSL1, optionally the inferring is further based on expression levels of at least one target gene, e.g. one, two, three, four, five, six, seven, eight, nine, ten eleven twelve or more target gene(s), of the HH pathway measured in the sample selected from the group comprising or consisting of: BCL2, FOXA2, FOXF1, H19, HHIP, IL1R2, JAG2, JUP, MIF, MYLK, NKX2.2, NKX2.8, PITRM1 and TOM1;
inferring activity of a AR cellular signaling pathway in the sample based at least on expression levels of three or more, e.g. three, four five six seven, eight, nine, ten eleven, twelve or thirteen, target gene(s) of the AR pathway measured in the sample selected from the group comprising or consisting of: KLK2, PMEPA1, TMPRSS2, NKX3_1, ABCC4, KLK3, FKBP5, ELL2, UGT2B15, DHCR24, PPAP2A, NDRG1, LRIG1, CREB3L4, LCP1, GUCY1A3, AR and EAF2, optionally the inferring is further based on expression levels of at least one target gene, e.g. one, two, three, four, five, six, seven, eight, nine, ten eleven twelve or more target gene(s), of the AR pathway measured in the sample selected from the group comprising or consisting of: APP, NTS, PLAU, CDKN1A, DRG1, FGF8, IGF1, PRKACB, PTPN1, SGK1 and TACC2;
inferring activity of a PI3K cellular signaling pathway in the sample based at least on expression levels of three or more, e.g. three, four five six seven, eight, nine, ten eleven, twelve or thirteen, target gene(s) of the PI3K pathway measured in the sample selected from the group comprising or consisting of: AGRP, BCL2L11, BCL6, BNIP3, BTG1, CAT, CAV1, CCND1, CCND2, CCNG2, CDK 1A, CDK 1B, ESR1, FASLG, FBX032, GADD45A, INSR, MXIl, NOS3, PCKl, POMC, PPARGCIA, PRDX3, RBL2, SOD2 and TNFSF10, optionally the inferring is further based on expression levels of at least one target gene, e.g. one, two, three, four, five, six, seven, eight, nine, ten eleven twelve or more target gene(s), of the PI3K pathway measured in the sample selected from the group comprising or consisting of: ATP8A1, C10orf10, CBLB, DDB1, DYRK2, ERBB3, EREG, EXT1, FGFR2, IGF1R, IGFBP1, IGFBP3, LGMN, PPM ID, SEMA3C, SEPP1, SESN1, SLC5A3, SMAD4 and TLE4, optionally the inferring is further based on expression levels of at least one target gene, e.g. one, two, three, four, five, six, seven, eight, nine, ten eleven twelve or more target gene(s), of the PI3K pathway measured in the sample selected from the group comprising or consisting of: ATG14, BIRC5, IGFBP1, KLF2, KLF4, MYOD1, PDK4, RAG1, RAG2, SESN1, SIRT1, STK11 and TXNIP, preferably inferring the activity of the FOXO/PI3K cellular signaling pathway in the sample is based at least on expression levels of at least three, target gene(s) of the FOXO/PI3K cellular signaling pathway measured in the extracted sample of the medical subject selected from the group consisting of: AGRP, BCL2L11, BCL6, BNTP3, BTG1, CAT, CAV1, CCND1, CCND2, CCNG2, CDKN1A, CDKN1B, ESR1, FASLG, FBX032, GADD45A, INSR, MXI1, NOS3, PCKl, POMC, PPARGC1A, PRDX3, RBL2, SOD2 and TNFSF10 and/or wherein inferring the oxidative stress state of the FOXO transcription factor element is based on the expression levels of one or more, preferably all of the target genes of a FOXO transcription factor SOD2, BNIP3, MXI1 and PCK1 measured in the extracted sample of the medical subject;
inferring activity of a TGFbeta cellular signaling pathway in the sample based at least on expression levels of three or more, e.g. three, four five six seven, eight, nine, ten eleven, twelve or thirteen, target gene(s) of the TGFbeta pathway measured in the sample selected from the group comprising or consisting of: ANGPTL4, CDC42EP3, CDKNIA, CDKN2B, CTGF, GADD45A, GADD45B, HMGA2, ID1, IL11, SERPINE1, INPP5D, JUNB, MMP2, MMP9, NKX2-5, OVOL1, PDGFB, PTHLH, SGK1, SKIL, SMAD4, SMAD5, SMAD6, SMAD7, SNAI1, SNAI2, TIMP1, and VEGFA, preferably, from the group consisting of: ANGPTL4, CDC42EP3, CDKNIA, CTGF, GADD45A, GADD45B, HMGA2, ID1, IL11, JUNB, PDGFB, PTHLH, SERPINE1, SGK1, SKIL, SMAD4, SMAD5, SMAD6, SMAD7, SNAI2, VEGFA, more preferably, from the group consisting of: ANGPTL4, CDC42EP3, CDKNIA, CTGF, GADD45B, ID1, IL11, JUNB, SERPINE1, PDGFB, SKIL, SMAD7, SNAI2, and VEGFA, more preferably, from the group consisting of: ANGPTL4, CDC42EP3, ID1, IL11, JUNB, SERPINE1, SKIL, and SMAD7;
inferring activity of a NFkB cellular signaling pathway in the sample based at least on expression levels of three or more, e.g. three, four five six seven, eight, nine, ten eleven, twelve or thirteen, target gene(s) of the NFkB pathway measured in the sample selected from the group comprising or consisting of: BCL2L1, BIRC3, CCL2, CCL3, CCL4, CCL5, CCL20, CCL22, CX3CL1, CXCL1, CXCL2, CXCL3, ICAM1, IL1B, IL6, IL8, IRF1, MMP9, NFKB2, NFKBIA, NFKB IE, PTGS2, SELE, STAT5A, TNF, TNFAIP2, TNIP1, TRAF1, and VCAM1;
inferring activity of a JAK-STAT1/2 cellular signaling pathway in the sample based at least on expression levels of three or more, e.g. three, four five six seven, eight, nine, ten eleven, twelve or thirteen, target gene(s) of the JAK-STAT1/2 pathway measured in the sample selected from the group comprising or consisting of: BID, GNAZ, IRF1, IRF7, IRF8, IRF9, LGALS1, NCF4, NFAM1, OAS1, PDCD1, RAB36, RBX1, RFPL3, SAMM50, SMARCB1, SSTR3, ST13, STAT1, TRMT1, UFDIL, USP18, and ZNRF3, preferably, from the group consisting of: IRF1, IRF7, IRF8, IRF9, OAS1, PDCD1, ST13, STAT1, and USP18.
inferring activity of a JAK-STAT3 cellular signaling pathway in the sample based at least on expression levels of three or more, e.g. three, four five six seven, eight, nine, ten eleven, twelve or thirteen, target gene(s) of the JAK-STAT3 pathway measured in the sample selected from the group comprising or consisting of: AKT1, BCL2, BCL2L1, BIRC5, CCND1, CD274, CDKN1A, CRP, FGF2, FOS, FSCN1, FSCN2, FSCN3, HIF1A, HSP90AA1, HSP90AB1, HSP90B1, HSPA1A, HSPA1B, ICAM1, IFNG, IL10, JunB, MCL1, MMP1, MMP3, MMP9, MUC1, MYC, NOS2, POU2F1, PTGS2, SAA1, STAT1, TIMP1, TNFRSF1B, TWIST1, VIM, and ZEB1, preferably, either from the group consisting of: BCL2L1, BIRC5, CCND1, CD274, FOS, HIF1A, HSP90AA1, HSP90AB1, MMP1, and MYC, or from the group consisting of: BCL2L1, CD274, FOS, HSP90B1, HSPA1B, ICAM1, IFNG, JunB, PTGS2, STAT1, TNFRSF1B, and ZEB1;
inferring activity of a MAPK-AP1 cellular signaling pathway in the sample based at least on expression levels of three or more, e.g. three, four five six seven, eight, nine, ten eleven, twelve or thirteen, target gene(s) of the MAPK-AP1 pathway measured in the sample selected from the group comprising or consisting of: BCL2L11, CCND1, DDIT3, DNMT1, EGFR, ENPP2, EZR, FASLG, FIGF, GLRX, IL2, IVL, LOR, MMP1, MMP3, MMP9, SERPINE1, PLAU, PLAUR, PTGS2, SNCG, TIMP1, TP53, and VIM, preferably, from the group consisting of: CCND1, EGFR, EZR, GLRX, MMP1, MMP3, PLAU, PLAUR, SERPINE1, SNCG, and TIMP1;
inferring activity of a Notch cellular signaling pathway in the sample based at least on expression levels of three or more, e.g. three, four five six seven, eight, nine, ten eleven, twelve or thirteen, target gene(s) of the Notch pathway measured in the sample selected from the group comprising or consisting of: CD28, CD44, DLGAP5, DTX1, EPHB3, FABP7, GFAP, GIMAP5, HES1, HES4, HES5, HES7, HEY1, HEY2, HEYL, KLF5, MYC, NFKB2, NOX1, NRARP, PBX1, PIN1, PLXND1, PTCRA, SOX9, and TNC, preferably, wherein two or more, for example, three, four, five, six or more, Notch target genes are selected from the group consisting of: DTX1, HES1, HES4, HES5, HEY2, MYC, NRARP, and PTCRA, and one or more, for example, two, three, four or more, Notch target genes are selected from the group consisting of: CD28, CD44, DLGAP5, EPHB3, FABP7, GFAP, GIMAP5, HES7, HEY1, HEYL, KLF5, NFKB2, NOX1, PBX1, PIN1, PLXND1, SOX9, and TNC;
inferring activity of a PR cellular signaling pathway in the sample based at least on expression levels of three or more, e.g. three, four five six seven, eight, nine, ten eleven, twelve or thirteen, target gene(s) of the PR pathway measured in the sample selected from the group comprising or consisting of: AGRP, BCL2L11, BCL6, BNIP3, BTG1, CAT, CAV1, CCND1, CCND2, CCNG2, CDKN1A, CDKN1B, ESR1, FASLG, FBXO32, GADD45A, INSR, MXI1, NOS3, PCKl, POMC, PPARGC1A, PRDX3, RBL2, SOD2 and TNFSF10, preferably selected from the group comprising or consisting of: BCL2L11, BCL6, BNIP3, BTG1, CAT, CAV1, CCNG2, FBXO32, GADD45A, INSR, MXIl, SOD2 and TNFSF10.

Herein, a FOXO transcription factor (TF) element is defined to be a protein complex containing at least one of the FOXO TF family members, i.e., FOXO1, FOXO3A, FOXO4 and FOXO6, which is capable of binding to specific DNA sequences, thereby controlling transcription of target genes.

Herein, a Wnt transcription factor (TF) element is defined to be a protein complex containing at least one of the TCF/LEF TF family members, i.e., TCF1, TCF3, TCF4 or LEF1, preferably wherein the Wnt TF element comprises beta-catenin/TCF4, which is capable of binding to specific DNA sequences, thereby controlling transcription of target genes.

Herein, a HH transcription factor (TF) element is defined to be a protein complex containing at least one of the GLI TF family members, i.e., GLI1, GLI2 or GLI3 which is capable of binding to specific DNA sequences, thereby controlling transcription of target genes.

Herein, an AR transcription factor (TF) element is defined to be a protein complex containing at least one or preferably a dimer of nuclear Androgen receptor.

Herein, an ER transcription factor (TF) element is defined to be a protein complex containing at least one or preferably a dimer of nuclear Estrogen receptor, preferably an ERalpha dimer.

Herein, the term "TGFbeta transcription factor element" or "TGFbeta TF element" or "TF element" when referring to the TGFbeta pathway is defined to be a protein complex containing at least one or, preferably, a dimer of the TGFbeta members (SMAD 1 , SMAD2, SMAD3 , SMAD5 and SMAD8 with SMAD4) or a trimer (two proteins from SMAD1, SMAD2, SMAD3, SMAD5 and SMAD8 with SMAD4), which is capable of binding to specific DNA sequences, thereby controlling transcription of target genes. Preferably, the term refers to either a protein or protein complex transcriptional factor triggered by the binding of TGFbeta to its receptor or an intermediate downstream signaling agent between the binding of TGFbeta to its receptor and the final transcriptional factor protein or protein complex. For example, it is known that TGFbeta binds to an extracellular TGFbeta receptor that initiates an intracellular "SMAD" signaling pathway and that one or more SMAD proteins (receptor-regulated or R-SMADs (SMAD1, SMAD2, SMAD 3, SMAD5 and SMAD8) and SMAD4) participate in, and may form a hetero-complex which participates in, the TGFbeta transcription signaling cascade which controls expression.

Herein, an NFkB transcription factor (TF) element is defined to be a protein complex containing at least one or, preferably, a dimer of the NFkB members (NFKB 1 or p50/pl05, NFKB2 or p52/p100, RELA or p65, REL, and RELB), which is capable of binding to specific DNA sequences, thereby controlling transcription of target genes.

Herein, the term "Notch transcription factor element" or "Notch TF element" or "TF element" is defined to be a protein complex containing at least the intracellular domain of one of the Notch proteins (Notch1, Notch2, Notch3 and Notch4, with corresponding intracellular domains N1ICD, N2ICD, N3ICD and N4ICD), with a co-factor, such as the DNA-binding transcription factor CSL (CBF1/RBP-JK, SU(H) and LAG-1), which is capable of binding to specific DNA sequences, and preferably one co-activator protein from the mastermind- like (MAML) family (MAML1, MAML2 and MAML3), which is required to activate transcription, thereby controlling transcription of target genes. Preferably, the term refers to either a protein or protein complex transcriptional factor triggered by the cleavage of one of the Notch proteins (Notch1, Notch2, Notch3 and Notch4) resulting in a Notch intracellular domain (N1ICD, N2ICD, N3ICD and N4ICD). For example, it is known that DSL ligands (DLL1, DLL3, DLL4, Jagged1 and Jagged2) expressed on neighboring cells, bind to the extracellular domain of the Notch protein/receptor, initiating the intracellular Notch signaling pathway and that the Notch intracellular domain participates in the Notch signaling cascade which controls expression.

Herein, the term "JAK-STAT1/2 transcription factor element" or "JAK-STAT1/2 TF element" or "TF element" when referring to the JAK-STAT1/2 is defined to be a protein complex containing at least a STAT1-STAT2 heterodimer or a STAT1 homodimer, which is capable of binding to specific DNA sequences, preferably the ISRE (binding motif AGTTTC NTTCNC/T) or GAS (binding motif TTC/A NNG/TAA) response elements, respectively, thereby controlling transcription of target genes. Preferably, the term refers to either a protein or protein complex transcriptional factor that is formed by different stimuli such as IFNs triggered by the binding of the stimulating ligand to its receptor resulting in downstream signaling.

Herein, the term "JAK-STAT3 transcription factor element" or "JAK-STAT3 TF element" or "TF element" when referring to the JAK-STAT3 pathway is defined to be a protein complex containing at least a STAT3 homodimer, which is capable of binding to specific DNA sequences, preferably the response elements with binding motif CTGGGAA, thereby controlling transcription of target genes. Preferably, the term refers to either a protein or protein complex transcriptional factor triggered by the binding of STAT3 inducing ligands such as interleukin-6 (IL-6) and IL-6 family cytokines to its receptor or an intermediate downstream signaling agent between the binding the ligand to its receptor and the final transcriptional factor protein or protein complex.

Herein, the term "AP-1 transcription factor element" or "AP-1 TF element" or "TF element" when referring to the MAPK-AP1 pathway is defined to be a protein complex containing at least a member of the Jun (e.g. c-Jun, JunB and JunB) family and/or a member of the Fos (e.g. c-Fos, FosB, Fra-1 and Fra-2) family and/or a member of the ATF family and/or a member of the JDP family, forming e.g. Jun∼Jun or Jun∼Fos dimers, capable of binding to specific DNA sequences, preferably the response elements 12-0-Tetradecanoylphorbol-13-acetate (TPA) response element (TRE) with binding motif 5'-TGA G/C TCA-3' or cyclic AMP response element (CRE) with binding motif 5'-TGACGTCA-3', thereby controlling transcription of target genes. Preferably, the term refers to either a protein or protein complex transcriptional factor triggered by the binding of AP-1 inducing ligands, such as growth factors (e.g., EGF) and cytokines, to its receptor or an intermediate downstream signaling agent, or triggered by the presence of an AP-1 -activating mutation.

When used herein, an *"in vitro* or *ex vivo* method" refers to any method taking place outside of the human or animal body. The method may therefore be performed on a sample comprising stem cells. Non limiting examples of samples comprising stem cells are cell cultures, cell suspension culture, tissue cultures, organoids, or cells or tissues extracted from a human or animal. When used herein the term "stem cell" may also refer to multiple stem cells or a sample comprising stem cells, the terms are used interchangeably herein.

When used herein, the step of "determining the differentiation state of the stem cell based on the compared cellular signaling pathway activities" refers to a step wherein differentiation state of the stem cell is determined based on the pathway activities. Based on the compared cellular signaling pathway activities the differentiation state of the stem cell can be determined by evaluating whether the pathway activities meet the expected pathway activities or deviate from it. For example, when in the comparing step the comparison is made with an internal reference pathway activity to normalize the three or more pathway activities, the pathway activities can be evaluated based on known or expected pathway activities for the stem cell. E.g. Embryonic stem cells are known to have high Hedgehog and Notch activity and low FOXO activity, and in such case are considered pluripotent. The determined activities of the stem cell can be evaluated based on this knowledge whether these criteria (i.e. high Hedgehog and Notch activity and low FOXO activity) are met, partly met or not met at all. Based on this evaluation the differentiation state of the stem cell can be determined to be pluripotent (when the set criteria are met) or partly differentiated (when the criteria are partially or not met).

Alternatively and preferably, if in the comparison step the comparison is made with external reference signaling pathway activities, preferably wherein the reference signaling pathways are the same as the three or more signaling pathways, the differentiation state of the stem cell can be directly evaluated based on this comparison. E.g. in a reference stem cell or sample which is known to be pluripotent (e.g. is validated to be able to differentiate in each of the three germ layers), the pathway activities for each of HH, Notch and FOXO have been determined. The determined values for these pathway activities in the stem cell can now be compared to these values. If the values are equal or closely similar the stem cell can be assumed to be pluripotent. To determine whether a value is considered equal as the reference value (wherein the value is the numerical number obtained for the pathway activity), a cut-off value can be used. Alternatively, if pathway activities have been determined on multiple reference samples, the threshold can be set for example by the standard deviation (or multiple standard deviations), or based on an alternative statistical evaluation.

The comparison step may further comprise additional reference samples. E.g. it may be beneficial to include stem cells which are partially differentiated as a reference, so that the comparison step includes reference signaling pathway activities from a pluripotent (or multipotent) stem cell and reference signaling pathway activities from a partially differentiated stem cell. This may be even more beneficial when the stem cell may differentiate in different lineages (e.g. the three germ layers), including references for each of the partially differentiated lineages may allow to further discriminate if the stem cell is partially differentiated, in which lineage it is differentiated.

Therefore preferably the method comprises:
- comparing the at least three cellular signaling pathway activities in the stem cell with at least three or more reference cellular signaling pathway activity, more preferably the method comprises:
- comparing the at least three cellular signaling pathway activities in the stem cell with at least three or more reference cellular signaling pathway activity, wherein the three or more cellular signaling pathways and the three or more reference cellular signaling pathways are the same pathways;
wherein the differentiation state of the reference sample is known (e.g. pluripotent, multipotent or partially differentiated).

Even more preferably, multiple reference samples (e.g. two or more) are used each with known but different differentiation states.

When used herein the term pluripotent refers to a stem cell, e.g. an embryonic stem cell or an induced pluripotent stem cell, that is capable into differentiating into each of the three germ layers. The cell is said to be able to differentiate into a germ layer when it is capable of differentiating into a cell type or progenitor of a cell type that is typically associated with that germ layer. Examples of tissues or organs associated with endoderm are the pharynx, the esophagus, the stomach, the small intestine, the colon, the liver, the pancreas, the bladder, the epithelial parts of the trachea and bronchi, the lungs, the thyroid, and the parathyroid. Examples of tissues or organs associated with mesoderm are muscle (smooth and striated), bone, cartilage, connective tissue, adipose tissue, circulatory system, lymphatic system, dermis, genitourinary system, serous membranes, spleen and notochord. Examples of tissues or organs associated with the ectoderm are epidermis, hair, nails, lens of the eye, sebaceous glands, cornea, tooth enamel, the epithelium of the mouth and nose, peripheral nervous system, adrenal medulla, melanocytes, facial cartilage, dentin of teeth, brain, spinal cord, posterior pituitary, motor neurons, retina.

It is known in the art that stem cells capable of developing into a germ layer will generally only do so provided that the required conditions are met. Generally a specific combination growth factors and culture conditions are required.

When used herein a multipotent stem cell refers to a stem cell such as an organ stem that is capable of differentiating into different specialized cells. A multipotent stem cell is generally committed to a germ layer and therefore can only differentiate into cells of the same germ layer. Non limiting examples multipotent stem cells are mesenchymal stem cells, hematopoietic stem cells, blood cord stem cells, adipose tissue-derived stem cells, cardiac stem cells, vascular stem cells, intestinal crypt stem cells, neural progenitor cells.

When used herein, a stem cell is considered at least partially differentiated when either it has lost its ability to self-renew, i.e. give rise to a stem cell of the same type, or is displaying a decreased ability to differentiate into all potential cell types or lineages it normally can differentiate into, i.e. it becomes committed to a single or a subset of cell types or lineages.

In an embodiment of the first aspect of the invention, the reference cellular signaling pathway activity is determined in the stem cell and the comparing step is used to normalize the at least three cellular signaling pathway activities in the stem cell.

In an embodiment of the first aspect of the invention, the reference cellular signaling pathway activity is determined in one or more reference sample(s) and the comparing step is used to compare the signaling pathway activity or activities with a reference with a known differentiation state,
preferably wherein the comparing step is performed by comparing the at least three cellular signaling pathway activities in the stem cell with a reference library, the reference library comprising the at least three cellular signaling pathway activities determined in at least two reference samples. In a further preferred embodiment the at least three cellular signaling pathways in the reference library are the same pathways as the three or more pathways of which the activity is to be determined.

In an embodiment of the first aspect of the invention the three or more, cellular signaling pathway activities further comprise one or more cellular signaling pathway activities selected from the group consisting of the cellular signaling pathway activities of the PI3K-FOXO, AR, ER, PR, NFkB, AP1-MAPK, JAK-STAT1/2 and PR pathways. Therefore preferably the method is based on determining or the result of a determining of the activities of three or more cellular signaling pathways, such as three, four, five, six, seven, eight, nine, ten or more cellular signaling pathways comprising three or more cellular signaling pathways, e.g. three, four or five, selected form the group consisting of TGFbeta, Notch, JAK-STAT3, Hedgehog, and Wnt and one or more cellular signaling pathways, such as one, two three, four, five, six, seven or eight cellular signaling pathways selected form the group consisting of PI3K-FOXO, AR, ER, NFkB, AP1-MAPK, JAK-STAT1/2 and PR pathways.

As demonstrated in the examples and figures below the TGFbeta, Notch, JAK-STAT3, Hedgehog, and Wnt pathways are relevant in the processes of maintaining stem cell pluripotency (multipotency) and differentiation. Therefore to determine the differentiation state of the stem cell at least three, preferably four, more preferably all five of these pathways activities are determined. It may however be beneficial to additionally determine the pathway activities of one or more of PI3K-FOXO, AR, ER, NFkB, AP1-MAPK, JAK-STAT1/2 and PR as it may provide additional information.

In an embodiment of the first aspect of the invention the stem cell is an iPS cell, an embryonic stem cell, an organoid cell, an organ or tissue derived stem cell or a cancer stem cell. In certain embodiments the stem cell is an embryonic stem cell or an induced pluripotent stem cell. The method may for example be used to determine the pluripotency of an embryonic stem cell or an iPS.

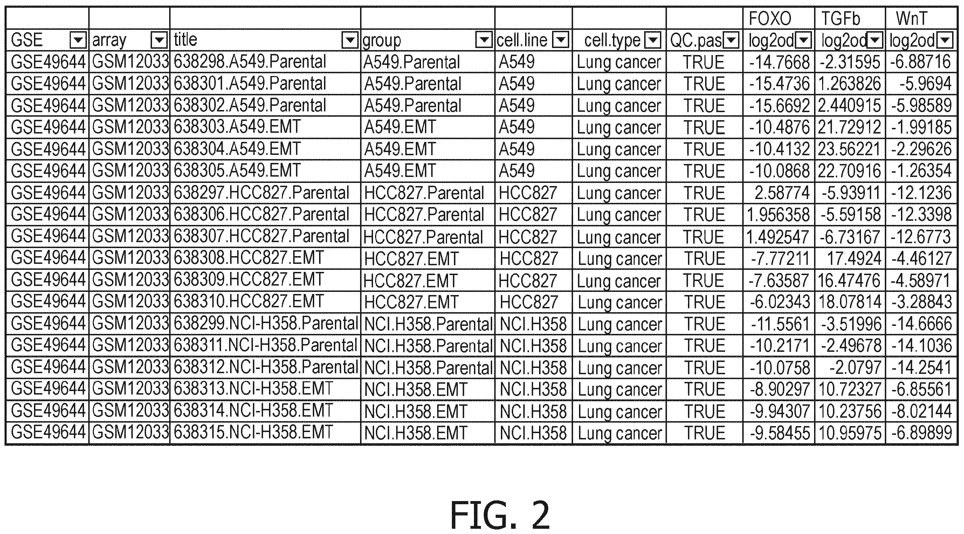

In certain embodiments the stem cell is a cancer stem cell. For example, Fig. 2 displays three different lung cancer cell lines which have or have not been differentiated to cancer stem cells by inducing endothelial-mesenchymal transition by treatment by supplementing TGFbeta. As can be seen in the figure, among the three cell lines consistently FOXO signaling is lower, and TGFbeta and Wnt signaling is higher in the cancer stem cells. This information can be utilized in the methods described herein. Therefore in a further embodiment, the state of a cancer stem cell is used to determine the sternness of the cancer stem cell, or more generally, the method is used to determine the sternness of a cancer cell. This may for example be achieved by creating two reference samples for the particular cancer type (e.g. lung cancer), one regular and one induce cancer stem cell reference, to determine pathways that can be utilized to distinguish cancer stem cells, and then performing the method of the invention by comparing the cancer cell or cancer stem cell with the reference pathway activities.

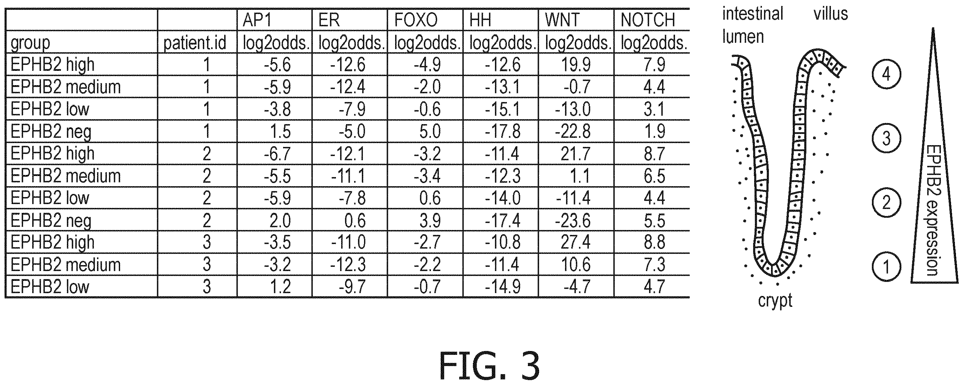

In certain embodiments the stem cell is an organ stem cell, e.g. an intestinal crypt stem cell. For example Fig. 3 displays the MAPK-AP1, ER, FOXO, HH, Wnt and Notch signaling pathway activities of different compartments of the intestinal crypt, indicated by EPHB2 expression. Here, high EPHB2 is indicative of the compartment comprising the stem cells, which self-renew and give rise to more differentiated daughter cells which slowly move towards the intestinal lumen while differentiating. The figure shows that when differentiating the stem cells increase MAPK-AP1, ER and FOXO signaling while decreasing HH, Wnt and Notch signaling. Therefore in a certain embodiment the stem cell is an organ stem cell, e.g. an intestinal crypt stem cell, and the method is used to determine the differentiation state. Reference samples representing the stem cell and various states of differentiation can be used as described herein. In a further embodiment the organ stem cell is an intestinal stem cell and the differentiation state is determined based on at least one, two or three pathways selected from MAPK-AP1, ER and FOXO and at least one, two or three pathways selected from HH, Wnt and Notch.

In an embodiment of the first aspect of the invention said method further comprises the step of determining the cellular signaling pathway activities of the three or more cellular signaling pathways. It is understood that the method may performed based on an input signal representing the cellular signaling pathway activity. Alternatively, the cellular signaling pathway may be determined as part of the method. The cellular signaling pathway activities may for example be determined based on the expression levels of target genes for the respective cellular signaling pathway. Expression levels may be used to determine the cellular signaling pathway activity as described herein.

In a preferred embodiment of the first aspect of the invention said method further comprises the step of determining the expression levels for three or more target genes for each cellular signaling pathway, and wherein said three or more cellular signaling pathway activities are determined based on said three or more expression levels of the target genes for each cellular signaling pathway. In an embodiment the signaling pathway measurements are performed using qPCR, multiple qPCR, multiplexed qPCR, ddPCR, RNAseq, RNA expression array or mass spectrometry. For example, a gene expression microarray data, e.g. Affymetrix microarray, or RNA sequencing methods, like an Illumina sequencer, can be used.

In a preferred embodiment of the first aspect of the invention the stem cell is an embryonic stem cell, and
wherein the differentiation state of the embryonic stem cell is determined to be pluripotent when three or more, preferably four, more preferably five, of the following criteria are met:
- TGFbeta signaling is high;
- Notch signaling is high;
- STAT3 signaling is high;
- Hedgehog signaling is high;
- Wnt signaling is low, or
wherein the differentiation state of the embryonic stem cell is determined to be at least partially differentiated when less than three of the above criteria are met or when at least three of the above criteria are not met.

The pathway activities may be determined with respect to reference samples with a known state. For example, a validated pluripotent stem cells sample may be used as a reference sample to determine the TGFbeta, Notch STAT3 HH and Wnt signaling pathway activities. In such way a "high TGFbeta signaling activity" may be defined as equal or comparable as the pathway activity obtained in the reference sample. Comparable activity in this context may be the activity plus or minus a preset cutoff value. Alternatively this may be set based on a statistical analysis, e.g. when multiple reference samples are analyzed the average value plus or minus the standard deviation. These same can be applied to the other pathways. Further, a deviating reference may be included (e.g. a stem cell validated to be not pluripotent or partially differentiated) to further define the values and boundaries between pluripotent and not pluripotent. E.g. if the numerical value for a pathway in the pluripotent reference is 20 and the value for the same pathway in the non-pluripotent reference is 6, the cutoff can be determined as 13 (6 + (20 -6)/2 = 13), meaning pathway activities with a value above 13 are considered belonging to a pluripotent state and below belonging to a non-pluripotent state.

In a preferred embodiment of the first aspect of the invention the stem cell is an induced pluripotent stem cell, and
wherein the differentiation state of the induced pluripotent stem cell is determined to be pluripotent when three or more, preferably four, more preferably five, of the following criteria are met:
- TGFbeta signaling is high;
- Notch signaling is high;
- STAT3 signaling is high;
- Hedgehog signaling is high;
- Wnt signaling is low, or
wherein the differentiation state of the induced pluripotent stem cell is determined to be at least partially differentiated when less than three of the above criteria are met.

The pathway activities may be determined with respect to reference samples with a known state as described above.

In a preferred embodiment of the first aspect of the invention the stem cell is an organ stem cell, and
wherein the differentiation state of the organ stem cell is determined to be multipotent when three or more of the following criteria are met:
- Notch signaling is high;
- Wnt signaling is high;
- Hedgehog signaling is low;
- MAPK signaling is low;
- ER signaling is low, or
wherein the differentiation state of the organ stem cell is determined to be partially differentiated when less than three of the above criteria are met,
preferably wherein the organ stem cell is an intestinal stem cell.

The pathway activities may be determined with respect to reference samples with a known state as described above.

In a preferred embodiment of the first aspect of the invention the cellular signaling pathway activities used to determine the stem cell differentiation state are used to determine the pluripotency or multipotency of the stem cell, wherein the pluripotency or multipotency of the stem cell is further used for one or more of:
- predicting the ability of the stem cell to maintain sternness and/or maintain a stem cell phenotype; and/or
- predicting the ability of the stem cell to give rise to differentiate to any of ectodermal, endodermal and mesodermal lineages or cell or tissue types thereof, and/or
- predicting the ability of the stem cell to maintain a balance between quiescence, proliferation, and regeneration, and/or
wherein the determining the differentiation state of the stem cell further comprising determining the lineage of the stem cell, preferably
wherein the lineage is selected from the group consisting of: embryonic stem cells (undifferentiated), anterior primitive streak, mesoderm, definitive endoderm, anterior foregut, posterior foregut, midgut/hindgut, pluripotent stem cells, multipotent stem cells, organ stem cells, intestinal stem cells, neural progenitors, terminally differentiated cells, and/or
wherein the method is used to control the quality of the stem cell, by comparing the three or more cellular signaling pathway activities with the three or more desired reference signaling pathway activities for that stem cell.

The pathway activities may be determined with respect to reference samples with a known state as described above.

In a preferred embodiment of the first aspect of the invention the stem cell is an embryonic stem cell or an induced pluripotent stem cell, and wherein the differentiation state of the embryonic stem cell or the induced pluripotent stem cell is determined using the following criteria:
- the stem cell is determined to be a pluripotent embryonic stem cell if FOXO signaling is very low, TGFbeta signaling is medium, Wnt signaling is very low and STAT3 signaling is high;
- the stem cell is determined to be a primitive streak stem cell if FOXO signaling is very low, TGFbeta signaling is low, Wnt signaling is low and STAT3 signaling is high;
- the stem cell is determined to be a definitive endoderm stem cell if FOXO signaling is low, TGFbeta signaling is low, Wnt signaling is very low and STAT3 signaling is low;
- the stem cell is determined to be a mesoderm stem cell if FOXO signaling is low, TGFbeta signaling is medium, Wnt signaling is low and STAT3 signaling is low;
- the stem cell is determined to be an anterior foregut stem cell if FOXO signaling is low, TGFbeta signaling is very low, Wnt signaling is very low and STAT3 signaling is high;
- the stem cell is determined to be an posterior foregut stem cell if FOXO signaling is low, TGFbeta signaling is low, Wnt signaling is very low and STAT3 signaling is high;
- the stem cell is determined to be an mid/hindgut stem cell if FOXO signaling is very low, TGFbeta signaling is very low, Wnt signaling is high and STAT3 signaling is high.

The pathway activities may be determined with respect to reference samples with a known state as described above.

In a second aspect, the invention relates to a method for generating a reference library, for use in a method for characterizing a stem cell based on the activity of three or more cellular signaling pathway activities, the method comprising:
obtaining two or more reference samples with known pluripotency or differentiation state;
- determining the activities of three or more cellular signaling pathways in the two or more samples;
wherein the two or more reference samples are stem cells, and wherein the two or more reference samples differ in at least one differentiation or pluripotency parameter.

Therefore when used herein a reference library refers to a set of numerical values representing the pathways activities of three or more cellular signaling pathways as obtained in one or more stem cells. Preferably the reference library is generated using multiple stem cells. Preferably the stem cell or stem cells used for generating the reference library have been characterized, i.e. have a known pluripotency, multipotency or differentiation state.

Preferably the three or more cellular signaling pathways are selected from the groups consisting of TGFbeta, Notch, JAK-STAT3, Hedgehog, and Wnt.

In a further preferred embodiment the three or more cellular signaling pathways further comprise one or more cellular signaling pathway activities selected from the group consisting of the cellular signaling pathway activities of the PI3K-FOXO, AR, ER, PR, NFkB, AP1-MAPK, JAK-STAT1/2 and PR pathways. Therefore preferably the method is based on determining the activities of three or more cellular signaling pathways, such as three, four, five, six, seven, eight, nine, ten or more cellular signaling pathways comprising three or more cellular signaling pathways, e.g. three, four or five, selected form the group consisting of TGFbeta, Notch, JAK-STAT3, Hedgehog, and Wnt and one or more cellular signaling pathways, such as one, two three, four, five, six, seven or eight cellular signaling pathways selected form the group consisting of PI3K-FOXO, AR, ER, PR, NFkB, AP1-MAPK, JAK-STAT1/2 and PR pathways.

Therefore, the comparing step in the method according to the first aspect of the invention is preferably performed using a reference library as described herein or as obtained or obtainable according to the method of the second aspect of the invention.

Preferably stem cells are used for the reference library with a known state, e.g. stem cell that have been validated to be pluripotent, multipotent, unipotent, partialy differentiated, etc. It may be further advantageous to use multiple stem cells with the same (or very similar) state in the reference library, multiple stem cells each with different but known states, or groups of stem cells where each group differs from each other (with respect of the state of the stem cell) and the cells within a group have the same (or very similar) state.

In a third aspect the invention relates to a non-transitory storage medium for characterizing a stem cell, storing instructions that are executable by a digital processing device to perform the method of the first aspect of the invention.

The non-transitory storage medium may be a computer-readable storage medium, such as a hard drive or other magnetic storage medium, an optical disk or other optical storage medium, a random access memory (RAM), read only memory (ROM), flash memory, or other electronic storage medium, a network server, or so forth. The digital processing device may be a handheld device (e.g., a personal data assistant or smartphone), a notebook computer, a desktop computer, a tablet computer or device, a remote network server, or so forth.

In a fourth aspect of the invention is provided a computer program determining the differentiation state of a stem cell, comprising program code means for causing digital processing device to perform the method of the first and/or the second aspect of the invention and the various embodiments thereof, when the computer program is run on a digital processing device. The computer program may be stored/distributed on a suitable medium, such as an optical storage medium or a solid-state medium, supplied together with or as part of other hardware, but may also be distributed in other forms, such as via the Internet or other wired or wireless telecommunication systems.

In a fifth aspect of the invention is provided a kit of parts comprising components for inferring activity of three or more cellular signaling pathways by determining the expression levels of three or more sets of target genes of the respective cellular signaling pathways,
wherein said components are polymerase chain reaction primers and probes directed to three or more target genes for each of the three or more cellular signaling pathway, and
wherein the three or more cellular signaling pathways comprise three or more cellular signaling pathways selected from the groups consisting of AR, ER, FOXO/PI3K, HH, NFkB, TGFbeta, WNT, NOTCH, AP1-MAPK, JAK-STAT1/2 and JAK-STAT3,
and optionally further comprising an apparatus comprising at least one digital processor configured to perform the method of the first aspect of the invention, the non-transitory storage medium according to the third aspect of the invention, and/or a computer program comprising program code means for causing a digital processing device to perform a method of the first aspect of the invention, when the computer program is run on the digital processing device.

In a sixth aspect of the invention is provided the use of the kit according to fifth aspect of the invention for performing the method according to the first aspect of the invention.

This application describes several preferred embodiments. Modifications and alterations may occur to others upon reading and understanding the preceding detailed description. It is intended that the application is construed as including all such modifications and alterations insofar as they come within the scope of the appended claims or the equivalents thereof.

Other variations to the disclosed embodiments can be understood and effected by those skilled in the art in practicing the claimed invention, from a study of the drawings, the disclosure, and the appended claims.

It shall be understood that the methods of the first aspect, the computer implemented invention of the second aspect, the apparatus of the third aspect, the non-transitory storage medium of fourth aspect, the computer program of the fifth aspect, the kits of the sixth aspect have similar and/or identical preferred embodiments, in particular, as defined in the dependent claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Calculations like the determination of the mortality risk performed by one or several units or devices can be performed by any other number of units or devices.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

It shall be understood that a preferred embodiment of the present invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

General: In all the figures where signal transduction pathway analysis scores are depicted, these are given as log2odds scores for pathway activity, derived from the probability scores for pathway activity provided by the Bayesian pathway model analysis. Log2odds scores indicate the level of activity of a signaling pathway on a linear scale.

Analyzed public datasets are indicated with their GSE number (in principle at the bottom of each figure), and individual samples with their GSM number (in principle most right column for clustering diagrams).

All validation samples for a signaling pathway model or an immune response/system model are independent samples and have not been used for calibration of the respective model to be validated.

Fig. 1. Human stem cell characterization in terms of signaling pathway activity. Shown are results of respectively PI3K-FOXO, Hedgehog (HH), TGFβ, Wnt, Notch, STAT3 pathway activities for individual samples; pathway activity scores are on a log 2odds scale. Activity of the FOXO transcription factor is measured as inverse readout for activity of the PI3K pathway. Individual sample annotation as provided in the public GEO database is on the left.
A. Human embryonic stem cells. GEO dataset GSE19902 containing sample data from a stem cell study. Study description: Pluripotent human embryonic stem cells (hES-T3) were cultured under different conditions, either on a mouse feeder layer (MEF) or with MEF-conditioned medium, or on a human feeder layer (T3HDF) or T3HDF-conditioned medium.
B. Various human iPS and HES cell lines from GEO dataset GSE17312, microarray data were generated within the BI Human Reference Epigenome Mapping Project.
C. Human bone marrow-derived mesenchymal stem cells, sample data from GEO dataset GSE84500.

Fig. 2. Cancer stem cell characterization with respect to signaling pathway activity. Signaling pathway analysis of Affymetrix U133Plus2.0 microarray data from a public dataset (GSE49644), containing sample data from a stem cell study described in Cancer Metab. 2014 Nov 3;2(1):20. Shown are results of respectively PI3K-FOXO, Hedgehog (HH), TGFβ, Wnt, Notch, STAT3 pathway activities for individual samples; pathway activity scores are on a log 2odds scale with colour coding ranging from blue (most inactive) to red (most active). Individual sample annotation as provided in the public GEO database is on the left. Study description: stimulation with TGFβ for a prolonged period (3 weeks) was used to induce epithelial-mesenchymal transition (EMT) in lung cancer cell lines A549, HCC827 and NCI-H358 cells to generate a cancer stem cell phenotype.

Fig. 3. A. Primary stem cell characterization with respect to signaling pathway activity. Signaling pathway analysis of Affymetrix U133Plus2.0 microarray data from a public dataset (GSE31255), containing sample data from a stem cell study described in Nature Medicine 2011; 17 (10): 1225-7). Normal human colonic mucosa epithelial cells from three different crypt-villus locations were isolated from colon biopsies from three individuals. Highest ephrin type-B receptor 2 (EPHB2) protein levels characterize intestinal stem cells (ISC). B. Intestinal crypt-villus structure, with decreasing EPHB2 expression from crypt compartment containing intestinal stem cells, to villus with differentiated mucosa cells. Sampling locations are indicated (roughly): 1, high EPHB2; 2, medium EPHB2; 3, low EPHB2; 4, negative EPHB2.

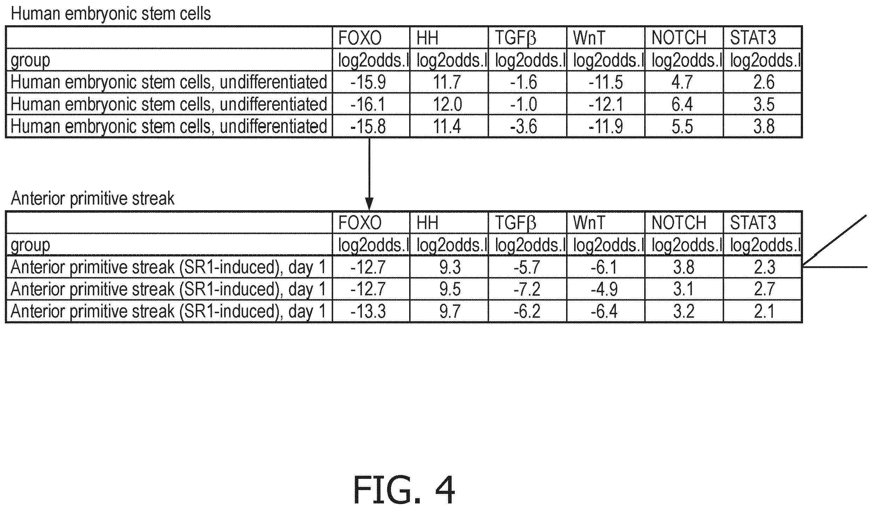

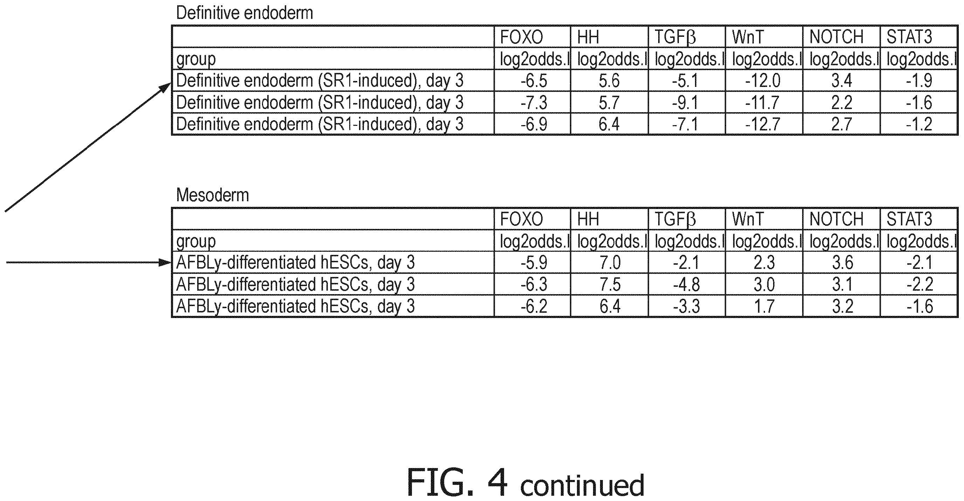

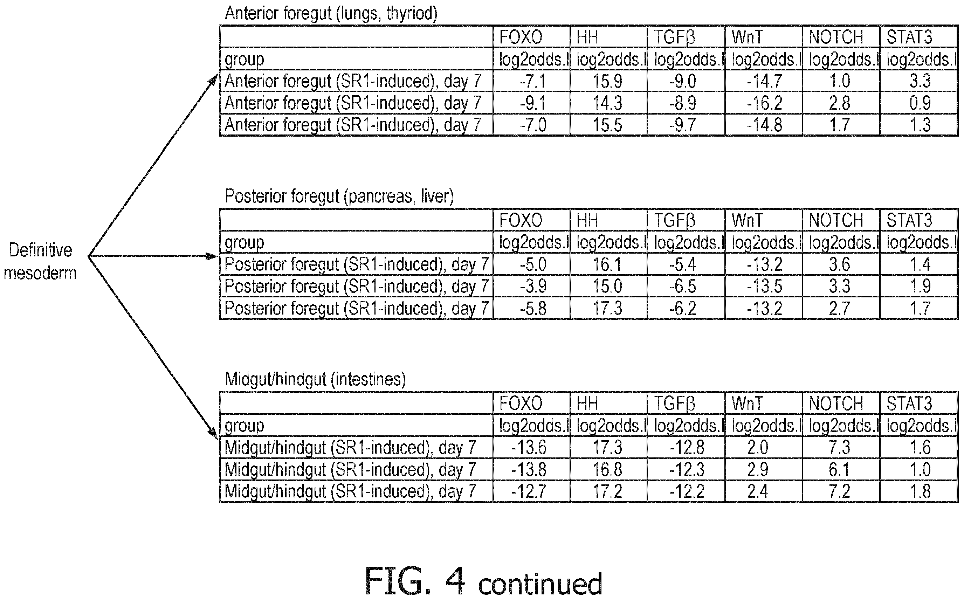

Fig. 4. Characterization of cells in various stages of differentiation in terms of multiple signaling pathway activity. Signaling pathway analysis of Affymetrix U133Plus2.0 microarray data from a public dataset (GSE52658), containing sample data from a stem cell study described in Cell Stem Cell. 2014 Feb 6;14(2):237-52. Using public mRNA expression datasets (GSE52658), we have measured combined pathway activity on the stem cells during different stages in the differentiation process. Loh et al. differentiated human embryonic stem cells (HES) into definitive endoderm and mesoderm, and subsequently to anterior foregut as precursor for lung and thyroid, to posterior foregut as precursor for pancreas and liver, and to mid/hindgut as precursor for intestinal tissue. HES cells were cultured under serum-free conditions in defined medium. To obtain anterior primitive streak cells (APS), ectoderm differentiation was excluded by activation of TGFbeta and Wnt pathway activity, while inhibiting the PI3K/mTOR pathway. The differentiation step from APS to definitive endoderm (DE) consisted of stimulation with high Activin and BMP blockade to prevent formation of mesoderm. Subsequently DE cells were differentiated during four days to different types of foregut cells (AFG/PFG/MHG), using treatment with BMP, Wnt, and FGF.

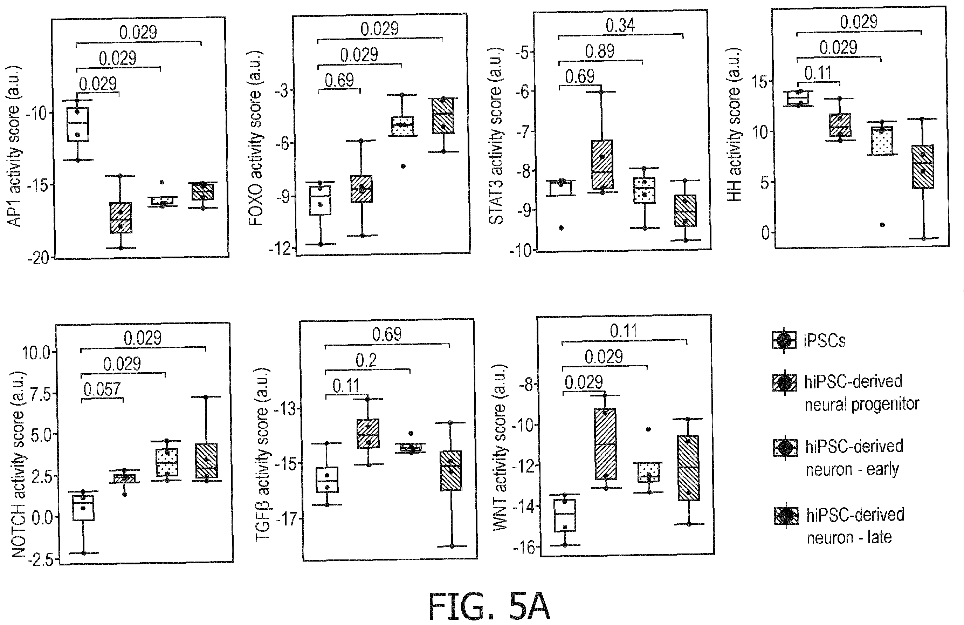

Fig. 5 A. Induced pluripotent stem cells (iPS): differentiation to neuronal cells is associated with loss of activity of pluripotency signaling pathways (MAPK, PI3K, HH), and gain of activity of neuronal progenitor signaling pathways (Notch, NFkB). Upon differentiation to neural progenitor cells, and differentiated (early and late) neurons, MAPK, JAK-STAT3, PI3K, HH pathway activity decreased, while activity of the neuron-specific Notch signaling pathway increased, together with NFkB pathway. Note: FOXO activity score is the inverse of PI3K pathway activity. Pathway Activity Scores are indicated as log2odds. Analyzed Affymetrix expression microarray data were from GEO dataset GSE74358. Unpublished results.

B. Upon differentiation to neural progenitor cells, and differentiated (early and late) neurons, MAPK, PI3K, HH pathway activity decreased, while activity of the neuron-specific Notch signaling pathway increased, together with NFkB pathway. Note: FOXO activity score is the inverse of PI3K pathway activity. Analyzed Affymetrix expression microarray data were from GEO dataset GSE74358. Unpublished results.

### EXAMPLES

Using the Gene Expression Omnibus (GEO) database (https://www.ncbi.nlm.nih.gov/gds/) Affymetrix HG-U133Plus2.0 datasets from clinical and preclinical studies were used. Information about the used datasets, sample type and and preparations can be found in Table 1 below. The literature references related to the original datasets are also included in the table. We used the pathway analysis to determine the signal transduction pathway activities (included pathways; AR, ER, PR, GR, HH, Notch, TGFbeta, WNT, JAK-STAT1/2, JAK-STAT3,NFkB, PI3K, MAPK). We compared the different pathway activities within the groups per dataset. Results per dataset can be found in the figure description.

Affymetrix U133Plus2.0 microarray data were downloaded from the public GEO database and signaling pathway analysis for PI3K-FOXO, Hedgehog (HH), TGFβ, Wnt, Notch, JAK-STAT, NFkB and MAPK-AP1 pathways was performed as described before, and presented as log 2 odds of the probability calculated by the respective signaling pathway models. Shown are results of respectively pathway activities for individual samples; pathway activity scores are on a log 2odds scale.

### Results

### A Signaling Pathway-basedpluripotency test.

Fig. 1 shows that signal transduction pathway analysis detects differences in activity in signal transduction pathways that are important for maintaining pluripotency. One of the envisioned utilities is to provide a quality control tool for pluripotent stem cell cultures. Results of multiple pathway analysis show that the Hedgehog (HH) pathway is always active; while activity of TGFβ, Notch and PI3K pathways is strongly dependent on culture conditions.

Pathway analysis can also be used to quantitatively characterize the sternness of various types of stem cells, as illustrated for cancer stem cells (Figure 2) and primary human intestinal stem cells that are present in the crypt compartment of the intestinal wall (Fig. 3).

Fig. 2: Results of multiple pathway analysis show higher TGFβ and Wnt pathway activity in EMT cells. PI3K pathway activity (the inverse of FOXO transcription factor activity) varies between cell lines. The increased activity of the signaling pathways TGFβ and Wnt confer stem cell renewal characteristics.

Fig. 3. In accordance with the stem cell character of these cells, the Wnt pathway was highly active, in combination with an active Notch pathway. The inactive FOXO transcription factor in these ISC indicates an active PI3K pathway. The low FOXO activity and high PI3K pathway activity are typically associated with dividing cells, and in agreement with the observed high KI67 expression. In contrast, low EPHB2 levels corresponded to differentiated, non-dividing cells.

### A Signaling Pathway-based test to quantitatively characterize differentiation steps and changes in pathway activity during differentiation.

Stem cells can be differentiated into various cell-types under the control of specific signals. Measurements of signaling pathway activity can be used to quantitatively assess the differentiation status of cultured cells, either at the final differentiation step, or during in-between differentiation steps, as illustrated by figure 4.

Fig. 4 We measured induction of Wnt activity, and reduced PI3K/mTOR pathway activity. The differentiation step from APS to definitive endoderm (DE) consisted of stimulation with high Activin and BMP blockade to prevent formation of mesoderm. We measured an increase in MAPK pathway activity, associated with reduced PI3K, STAT3 and Wnt pathway activity (in contrast to mesoderm). Subsequently DE cells were differentiated during four days to different types of foregut cells (AFG/PFG/MHG), using treatment with BMP, Wnt, and FGF. In contrast to the most anterior pharyngeal endoderm (by inhibition of BMP/Wnt/FGF pathways), the posterior mid/hindgut intestinal precursor cells showed higher Wnt pathway activity, associated with Notch, TGFβ and MAPK-AP1 activity.

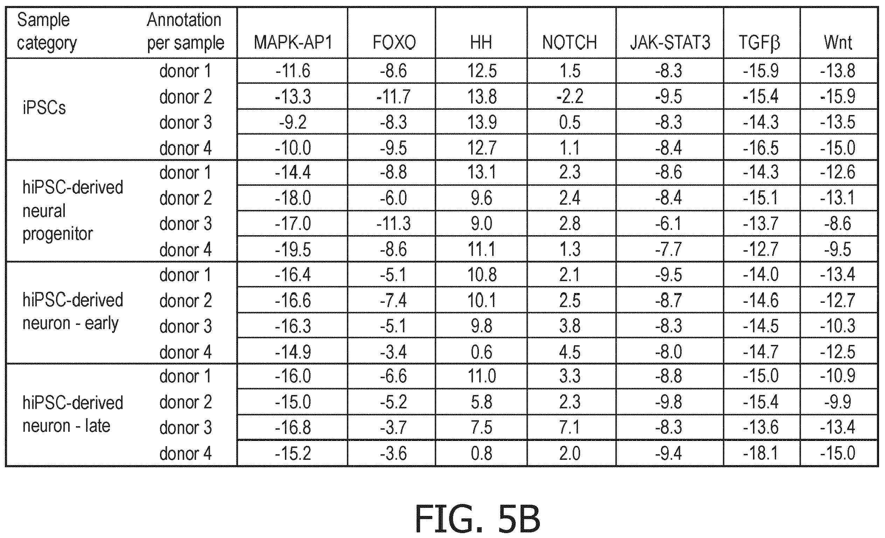

Differentiation of iPS cells to neural progenitor cells was associated with induction of FOXO activity, and therefore with reduced PI3K pathway activity, and with Notch pathway activity (Fig. 5).

A Signaling Pathway-based test to quantitatively characterize the differentiation potential towards mesodermal, endodermal and ectodermal lineages.

### Computational models for interpretation of pathway analysis results

### Computational model for interpretation of pathway analysis results to provide a quantitative score for pluripotency

### Linear model

The score consists of 1 point for each developmental stem cell pathway that should be active in pluripotent stem cells: PI3K pathway, Hedgehog pathway, TGFbeta pathway, Notch pathway, STAT3 pathway.

In the results of Fig. 1

| sample | PI3K pathway | Hedgehog pathway | TGFbeta pathway | Notch pathway | STAT3 pathway | Total score |
|---|---|---|---|---|---|---|
| 1 | 0 | 1 | 1 | 1 | 1 | 4 |
| 2 | 0 | 1 | 1 | 1 | 1 | 4 |
| 3 | 1 | 1 | 0 | 0 | 1 | 3 |
| 4 | 1 | 1 | 0 | 0 | 1 | 3 |
| 5 | 1 | 1 | 1 | 1 | 1 | 5 |
| 6 | 1 | 1 | 1 | 1 | 1 | 5 |
| 7 | 1 | 1 | 0 | 0 | 1 | 3 |
| 8 | 1 | 1 | 0 | 0 | 1 | 3 |

## Claims

1. An *in vitro* or *ex vivo* method for determining the differentiation state of a stem cell, based on determining, or the result of a determining of the activities of at least three cellular signaling pathways selected from the groups consisting of TGFbeta, Notch, JAK-STAT3, Hedgehog, and Wnt, the method comprising:
- comparing the at least three cellular signaling pathway activities in the stem cell with at least one reference cellular signaling pathway activity;
- determining the differentiation state of the stem cell based on the compared cellular signaling pathway activities, wherein the differentiation state of the stem cell is determined to be pluripotent, multipotent, unipotent, or at least partially differentiated.

2. *In vitro* or *ex vivo* method according to claim 1, wherein the reference cellular signaling pathway activity is determined in the stem cell and the comparing step is used to normalize the at least three cellular signaling pathway activities in the stem cell.

3. *In vitro* or *ex vivo* method according to claim 1, wherein the reference cellular signaling pathway activity is determined in one or more reference sample(s) and the comparing step is used to compare the signaling pathway activity or activities with a reference with a known differentiation state,
preferably wherein the comparing step is performed by comparing the at least three cellular signaling pathway activities in the stem cell with a reference library, the reference library comprising the at least three cellular signaling pathway activities determined in at least two reference samples.

4. *In vitro* or *ex vivo* method according to claim any one of the preceding claims, wherein the three or more, cellular signaling pathway activities further comprise one or more cellular signaling pathway activities selected from the group consisting of the cellular signaling pathway activities of the PI3K-FOXO, AR, ER, PR, NFkB, AP1-MAPK, JAK-STAT1/2 and PR pathways.

5. *In vitro* or *ex vivo* method according to any one of the preceding claims, wherein the stem cell is an iPS cell, an embryonic stem cell, an organoid cell, an organ or tissue derived stem cell or a cancer stem cell.

6. *In vitro* or *ex vivo* method according to any one of the preceding claims, wherein said method further comprises the step of determining the cellular signaling pathway activities of the three or more cellular signaling pathways.

7. *In vitro* or *ex vivo* method according to claim 6, wherein said method further comprises the step of determining the expression levels for three or more target genes for each cellular signaling pathway, and wherein said three or more cellular signaling pathway activities are determined based on said three or more expression levels of the target genes for each cellular signaling pathway.

8. Method according to any one of the preceding claims, wherein the stem cell is an embryonic stem cell, and
wherein the differentiation state of the embryonic stem cell is determined to be pluripotent when three or more, preferably four, more preferably five, of the following criteria are met:
- TGFbeta signaling is high;
- Notch signaling is high;
- STAT3 signaling is high;
- Hedgehog signaling is high;
- Wnt signaling is low, or
wherein the differentiation state of the embryonic stem cell is determined to be at least partially differentiated when less than three of the above criteria are met.

9. Method according to any one of claims 1 to 7, wherein the stem cell is an induced pluripotent stem cell, and
wherein the differentiation state of the induced pluripotent stem cell is determined to be pluripotent when three or more, preferably four, more preferably five, of the following criteria are met:
- TGFbeta signaling is high;
- Notch signaling is high;
- STAT3 signaling is high;
- Hedgehog signaling is high;
- Wnt signaling is low, or
wherein the differentiation state of the induced pluripotent stem cell is determined to be at least partially differentiated when less than three of the above criteria are met.

10. Method according any one of the preceding claims, wherein the stem cell is an organ stem cell, and
wherein the differentiation state of the organ stem cell is determined to be multipotent when three or more of the following criteria are met:
- Notch signaling is high;
- Wnt signaling is high;
- Hedgehog signaling is low;
- MAPK signaling is low;
- ER signaling is low, or
wherein the differentiation state of the organ stem cell is determined to be partially differentiated when less than three of the above criteria are met,
preferably wherein the organ stem cell is an intestinal stem cell.

11. In vitro or ex vivo method according to any one of the preceding claims, wherein the cellular signaling pathway activities used to determine the stem cell differentiation state are used to determine the pluripotency or multipotency of the stem cell, wherein the pluripotency or multipotency of the stem cell is further used for one or more of:
- predicting the ability of the stem cell to maintain sternness and/or maintain a stem cell phenotype; and/or
- predicting the ability of the stem cell to give rise to differentiate to any of ectodermal, endodermal and mesodermal lineages or cell or tissue types thereof, and/or
- predicting the ability of the stem cell to maintain a balance between quiescence, proliferation, and regeneration, and/or
wherein the determining the differentiation state of the stem cell further comprising determining the lineage of the stem cell, preferably
wherein the lineage is selected from the group consisting of: embryonic stem cells (undifferentiated), anterior primitive streak, mesoderm, definitive endoderm, anterior foregut, posterior foregut, midgut/hindgut, pluripotent stem cells, multipotent stem cells, organ stem cells, intestinal stem cells, neural progenitors, terminally differentiated cells, and/or
wherein the method is used to control the quality of the stem cell, by comparing the three or more cellular signaling pathway activities with the three or more desired reference signaling pathway activities for that stem cell.

12. Method for generating a reference library, for use in a method for characterizing a stem cell based on the activity of three or more cellular signaling pathway activities, the method comprising:
- obtaining two or more reference samples with known pluripotency or differentiation state;
- determining the activities of three or more cellular signaling pathways in the two or more samples;
wherein the two or more reference samples are stem cells, and wherein the two or more reference samples differ in at least one differentiation or pluripotency parameter.

13. A non-transitory storage medium for characterizing a stem cell, storing instructions that are executable by a digital processing device to perform the method of any of claims 1 to 11.

14. A kit of parts comprising components for inferring activity of three or more cellular signaling pathways by determining the expression levels of three or more sets of target genes of the respective cellular signaling pathways,
wherein said components are polymerase chain reaction primers and probes directed to three or more target genes for each of the three or more cellular signaling pathway, and
wherein the three or more cellular signaling pathways comprise three or more cellular signaling pathways selected from the groups consisting of AR, ER, FOXO/PI3K, HH, NFkB, TGFbeta, WNT, NOTCH, AP1-MAPK, JAK-STAT1/2 and JAK-STAT3,
and optionally further comprising an apparatus comprising at least one digital processor configured to perform the method of any one of claims 1 to 11, the non-transitory storage medium according to claim 13, and/or a computer program comprising program code means for causing a digital processing device to perform a method of any of claims 1 to 11, when the computer program is run on the digital processing device.

15. Use of the kit according to claim 14 for performing the method according to any one of claims 1 to 11.
